# EUROPEAN PATENT APPLICATION

(11) **EP 4 198 139 A1**
(43) Date of publication of application: **21.06.2023**
(21) Application number: 21214551.0
(22) Date of filing: 14.12.2021
(51) Int. Cl.: C12P 19/04, C12P 19/26, C12P 19/02, C12P 19/18, C12N 15/52, C12N 9/12, C12N 9/10, C12N 9/90, C12N 9/16, C12N 9/04

(54) **METHODS OF PRODUCTION OF HEPAROSAN, HEPARAN SULFATE AND HEPARIN IN YEAST**

(71) Applicant: Genhtis Fine Chemicals GmbH, 04769 Mügeln (DE)
(72) Inventor: KÖRNER, Johann Dietmar, 04769 Mügeln (DE)
(74) Representative: Prüfer & Partner mbB Patentanwälte · Rechtsanwälte

(57) **Abstract**

The present invention refers to recombinant yeast cells that produce an increased amount of UDP-D-glucuronic acid and/or UDP-N-acetyl-D-glucosamine; and optionally 3'-phospho-adenylylsulfate, when compared to its wildtype cells. The present invention further refers to a method of producing heparan sulfate, comprising a step of cultivating the recombinant yeast cell of the present invention, to the use of said invneitve recombinant yeast cell for producing heparan sulfate, and finally to heparan sulfates obtained when applying the method of the present invention.

## Description

### Field of the invention

The present invention refers to recombinant yeast cells that produce an increased amount of UDP-D-glucuronic acid and/or UDP-N-acetyl-D-glucosamine; and optionally 3'-phospho-adenylylsulfate, when compared to its wildtype cells. The present invention further refers to a method of producing heparan sulfate, comprising a step of cultivating the recombinant yeast cell of the present invention, to the use of said invneitve recombinant yeast cell for producing heparan sulfate, and finally to heparan sulfates obtained when applying the method of the present invention.

### Description of the background art

Heparin is a naturally occurring glycosaminoglycan stored in the secretory granules of mast cells. Even though its innate biological function is not entirely elucidated, heparin was discovered to be a potent anticoagulant drug in the 1910s. Its anticoagulant properties are attributed to heparin's high sulfation degree and the resulting negative charge, mediating factor Xa and thrombin binding to antithrombin. Heparin has, therefore, been a well proven and widely used medication for thrombosis for well over half a century.

As the annual global market size of heparin is estimated at about 7 billion USD in recent years, its volume is likely to increase substantially as new therapeutic applications for heparin and less sulfonated heparan sulfate analogs are currently established. Sulfation of the glycosaminoglycan in precise patterns modulates interactions with growth factors, enzymes, inhibitors, etc., enabling novel applications for these well-defined heparan-sulfates as anticancer, antimicrobial, anti-diabetic, anti-neurodegenerative and even better anticoagulant drugs.

Despite this apparent demand, the main source of heparin and its analogs remains animal tissue, in particular porcine intestines, which may lead to contamination of the drug and shortages during animal disease outbreaks. The unreliable nature of conventional heparin sourcing, the difficulties of synthetic heparin production, the large existing heparin market volume as well as the potential applications for novel heparan sulfates motivate efforts to develop microbial cell factories for recombinant biosynthesis of heparin and its analogs.

Even though it has not yet been shown previously, yeasts are potent cell factory candidates for heparin, heparan-sulfate and heparosan (the heparin and heparan sulfate precursor) production. While bacterial enzymes can be employed for heparosan synthesis, all enzymes required for heparosan modification, i.e. for heparan sulfate and heparin biosynthesis, are exclusively of vertebrate origin. Since enzymes of eukaryotic origin are generally more stable and functional when expressed in eukaryotic hosts, yeasts such as Saccharomyces cerevisiae (S. cerevisiae) are better suited for heparin biosynthesis than bacterial systems.

Despite clear advantages of yeasts as cell factories for heparosan, heparan-sulfate and heparin, three distinct, yet closely linked engineering challenges had to be overcome: First, to establish the yeast cell factory as a viable alternative to conventional heparin sourcing, it needs to prove cost efficient and result in high product titers. Thus, yeast chassis with efficient substrate utilisation and high precursor production had to be engineered (unit 1). Further, these novel efficient yeast strains had to be engineered to achieve heparosan production (unit 2). Finally, the heparosan producing strains had to be modified for the biosynthesis of heparin and other heparan sulfates with defined sulfation patterns (unit 3).

The solution for these hurdles, as presented here, not only results in a superior production platform for heparin, but also enables industrial scale production of novel heparan sulfates with a wide range of medical applications.

### Summary of the invention

### (1) Optimised Chassis Engineering

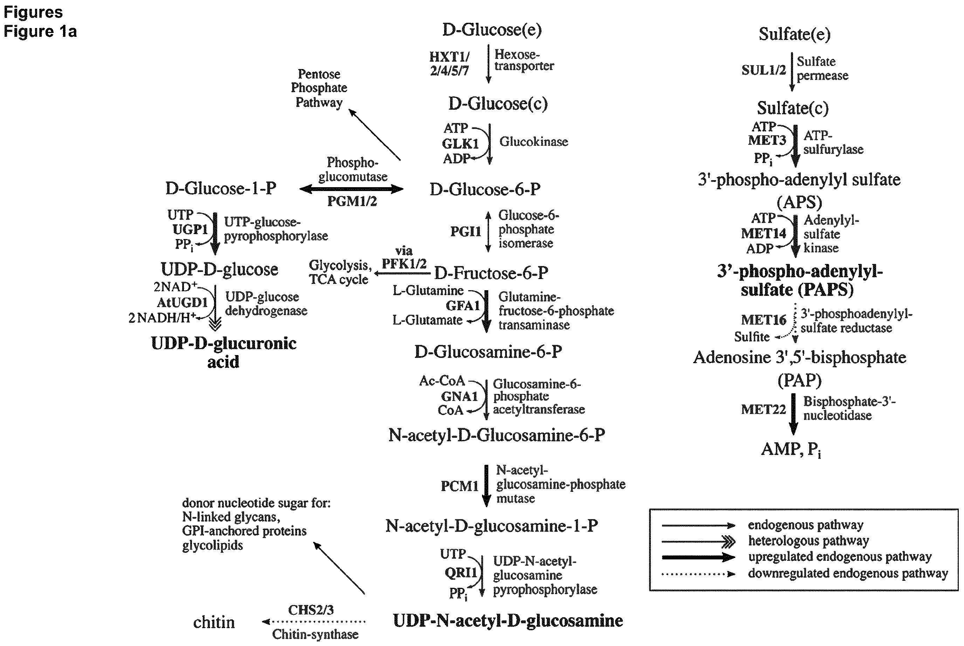

To establish yeast cell factories as economically viable production platforms for heparosan and heparan sulfates, it was crucial to engineer recombinant yeast chassis that efficiently utilize cheap substrates (such as D-glucose, (NH4)SO4) in order to provide high cytosolic precursor concentrations for heparosan synthesis (UDP-D-glucuronic acid, UDP-N-acetyl-D-glucosamine) and modification (3'-phospho-adenylylsulfate). The disjointed biosynthesis pathways for the UDP-sugars and 3'-phosphoadenylylsufate (PAPS) enabled separate optimisations in a modular pathway engineering approach as shown in Fig. 1a.

It was the scope of the first module of this modular pathway engineering approach to achieve high cytosolic concentrations of UDP-D-glucuronic acid (UDP-GlcA) and UDP-N-acetyl-D-glucosamine (UDP-GlcNAc) in a recombinant S. cerevisiae chassis. In contrast to UDP-GlcNAc, UDP-GlcA is not endogenously produced in S. cerevisiae. However, heterologous UDP-GlcA synthesis in the yeast has been previously accomplished through the integration of UDP-glucose dehydrogenase from Arabidopsis thaliana (AtUGD1) [1]. Therefore, the heterologous AtUGD1 was integrated into the genome of the S. cerevisiae S288C parent strain, yielding a recombinant strain capable of UDP-GlcNAc as well as UDP-GlcA production. Even though the cytosolic pool of UDP-glucose, the direct precursor of UDP-GlcA, is innately large in S. cerevisiae, the endogenous phosphoglucomutase PGM1/2 and UTP-glucose-1-phosphat uridylyltransferase (UTP-glucose pyrophosphorylase) UGP1, which directly lead to UDP-glucose from D-glucose-6-P, were additionally overexpressed in a subsequent strain to increase cytosolic UDP-GlcA concentration.

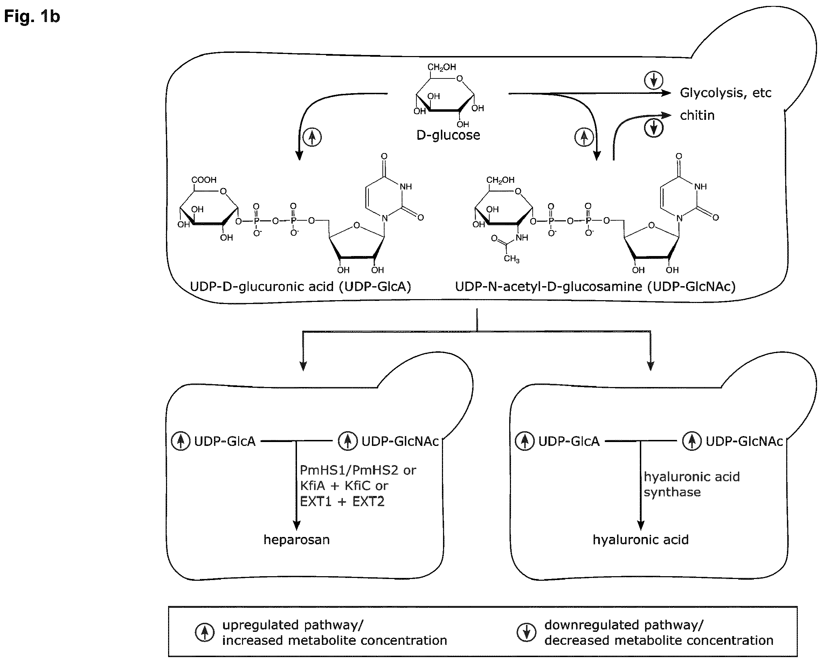

Mainly as a precursor of chitin, UDP-GlcNAc is synthesised in S. cerevisiae from fructose-6-P in four steps: Fructose-6-P is first transaminated (transaminase encoded by GFA1) to glucosamine-6-P utilising L-glutamine as nitrogen donor. Glucosamine-6-P is subsequently acetylated (acetylase encoded by GNA1) to N-acetyl-glucosamine-6-P and then converted to N-acetyl-glucosamine-1-P by a P-acetyl-glucosamine mutase encoded by PCM1. In the last synthesis step, N-acetyl-glucosamine-1-P is converted into UDP-GlcNAc by a UDP-GlcNAc pyrophosphorylase (encoded by QRI1). It has been shown that S. cerevisiae cultures treated with mating pheromones, which upregulate UDP-GlcNAc and consequently chitin production, lead to increased transcription of GFA1 and PCM1. This dynamic suggests that GFA1 and PCM1 are bottlenecks in the UDP-GlcNAc pathway. Therefore, the endogenous transaminase GFA1 and P-acetyl-glucosamine mutase PCM1 were overexpressed in a subsequent S. cerevisiae strain to yield an increased cytosolic UDP-GlcNAc concentration. To prevent consumption of cytosolic UDP-GlcNAc, the transcription of CHS3 encoding chitin synthase III was also downregulated, as chitin synthase III synthesises ca. 90% of chitin in S. cerevisiae. The downregulation was achieved by replacing the native CHS3 promoter with the weaker KEX2 promoter, thereby decreasing CHS3 transcription. The resulting yeast strain (which consists of the respective yeast cells) with increased cytosolic concentrations of UDP-GlcA and UDP-GlcNAc can serve as chassis for increased glycosaminoglycan (e.g. heparosan) production (Fig. 1b).

It was the objective of the second module of this modular pathway engineering approach to increase the cytosolic 3'-phospho-adenylylsulfate (PAPS) concentration in recombinant S. cerevisiae strains in addition to the previously improved UDP-sugar concentrations. To achieve that, recombinant S. cerevisiae strains were engineered to overexpress the sulfate adenylyltransferase (ATP-sulfurylase) MET3, the adenylyl-sulfate kinase MET14, and the adenosine 3',5'-bisphosphate nucleotidase MET22. Transcription of the endogenous 3'-phosphoadenylylsulfate reductase MET16 was downregulated to restrict PAPS consumption similar to an approach applied in Escherichia coli (E. coli) [2].

Overexpression and downregulation of a certain gene can be achieved by any suitable method that is known to a person skilled in the art. In the present invention, in both modules of the modular pathway engineering approach, i.e. the UDP-sugar module and the PAPS module, overexpression was achieved by chromosomal integration of an additional gene copy with a strong promoter (pTDH3, pTEF1, or pPGK1). Downregulation was achieved by exchanging the native promoter of the corresponding gene with the weak promoter of KEX2 (pKEX2).

### (2) Engineering of Heparosan Producing Yeast Strains

Heparosan, a glycosaminoglycan consisting of repeating disaccharide units (-GlcA-β1,4-GlcNAc-a1,4-), is the unmodified GAG precursor of heparin and other heparan sulfates. In mast cells, the biosynthesis of heparosan is initiated through the formation of a tetrasaccharide linkage region (-GlcA-β1,3-Gal-β1,3-Gal-β1,4-Xyl-O-Ser) on a specific Ser residue of a serglycin core protein. The formation of this linker is catalysed by four enzymes, each elongating the linker by one residue from the respective UDP-sugar. The tetrasaccharide linkage region serves as primer for the subsequent heparosan ([-GlcA-β1,4-GlcNAc-a1,4-]n) synthesis by alternating addition of glucuronate and N-acetyl-glucosamine residues from their UDP-sugars. This polymerisation is executed by a hetero-oligomeric complex of the GlcA/GlcNAc transferases II EXT1 and EXT2 from the exotosin family. This complex eukaryotic pathway for heparosan biosynthesis requiring a number of different enzymes and UDP-sugars can be replaced with bacterial heparosan synthases requiring only UDP-GlcA and UDP-GlcNAc. While E. coli K5 expresses two genes, KfiC and KfiA, encoding for a UDP-GlcA glucosyltransferase and a UDP-GlcNAc glucosyltransferase respectively, Pasteurella multocida (P. multocida) expresses two highly homologous dual action heparosan synthases, PmHS1 and PmHS2. Even though both P. multocida enzymes display glucuronic acid and hexosamine transfer activity (dual-action), their different catalytic preferences suggest distinct roles for heparosan synthesis: PmHS2 exhibits less dependency on polysaccharide primers while PmHS1 possesses a 3-fold faster heparosan elongation rate leading to larger molecular-weight polymers. When PmHS2 was heterologously expressed in Bacillus megaterium, heparosan titers of about 250 mg/L in shake flasks and 2.74 g/L in bioreactors were achieved [3].

In this work, genes encoding PmHS1 and PmHS2 were integrated into the genome of an engineered S. cerevisiae strain, capable of UDP-GlcA and UDP-GlcNAc biosynthesis, yielding recombinant yeast strains capable of heparosan production. However, it was observed that the bacterial enzymes (PmHS1 and PmHS2) tend to localize in the yeast nucleus, thereby impeding efficient heparosan biosynthesis. To counteract the nuclear localisation, the nuclear export sequence (NES) of S. cerevisiae's Hsp70 was fused to the C-termini of the heparosan synthases. When PmHS1_NES and PmHS2_NES were expressed in S. cerevisiae, improved heparosan production was observed, particularly for the PmHS1_NES expressing strain. Co-expression of PmHS1_NES and PmHS2_NES further improved heparosan titer, which is likely due to different characteristics of the isoforms: PmHS2 better synthesises heparosan primers while PmHS1 is the superior heparosan synthase for elongation. Co-expression of PmHS1_NES and PmHS2_NES in a heterologous S. cerevisiae optimized for UDP-GlcA and UDP-GlcNAc production (see Unit (1) "Optimised Chassis Engineering") resulted in the best heparosan producing strain.

### (3) Engineering Yeast Strains for the Production of Heparan Sulfates and Heparin

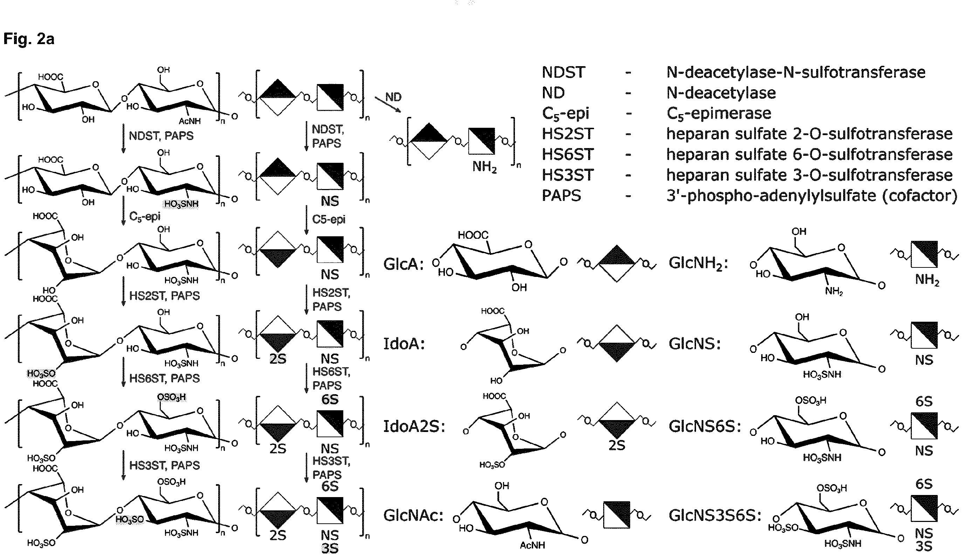

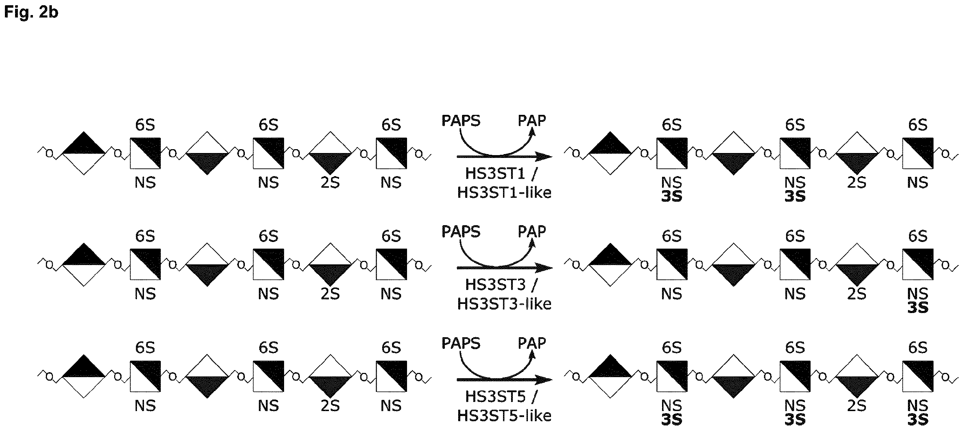

Following the biosynthesis of heparosan, modification of the polysaccharide by a number of enzymes yields many distinct biofunctional heparan sulfates (Fig. 2a). The two initial heparosan modification steps, N-deacetylation and PAPS-dependent N-sulfation of N-acetyl-glucosamine are carried out by the dual-action enzyme N-deacetylase-N-sulfotransferase (NDST). While four highly homologous vertebrate NDST isoforms are known, only NDST1 and NDST2 exhibit high N-deacetylation as well as N-sulfation activity. In contrast, isoform NDST3 shows high N-deacetylation but very low N-sulfation activity. The N-sulfated Glucosamine (GlcNS) is necessary for the subsequent C5-epimerization of the adjacent D-glucuronic acid (GlcA) residue to L-iduronic acid (IdoA) by C5-epimerase (C5-epi). Epimerization to L-iduronic acid increases the residue's affinity for heparan sulfate 2-O-sulfotransferase (HS2ST) 5-fold, promoting sulfate transfer from PAPS to the C2 of the L-idoronyl residue. In addition to heparosan modification by N-deacetylation, N-sulfation, C5-epimerization and 2-O-sulfation, three isoforms of heparan sulfate 6-O-sulfotransferases (HS6ST) have been reported to catalyse the sulfate transfer from PAPS to the C6 of glucosamine residues of heparosan. All three isoforms show a prominent preference for GlcNS/GlcNAc residues immediately downstream of IdoA residues. The final modification of the heparan sulfate, the sulfate transfer to the 3-OH of a glucosamine unit is rare in nature but crucial for many biological functions of the resulting GAG. The seven known heparan sulfate 3-O-sulfotransferase (HS3ST) isoforms can be classified by their ability to transfer a sulfate group to a glucosamine bound at its non-reducing end to either GlcA, IdoA, IdoA2S or a combination thereof (Fig. 2b). For instance, HS3ST isoform 1 (HS3ST1) catalyses the sulfate transfer exclusively to the 3-OH of glucosamines that are linked to either GlcA or IdoA. In contrast to HS3ST1, HS3ST3 requires an adjacent 2-O-sulfated IdoA (IdoA2S) residue for the sulfate transfer. HS3ST5's activity is independent of the linked uronic acid residues [4].

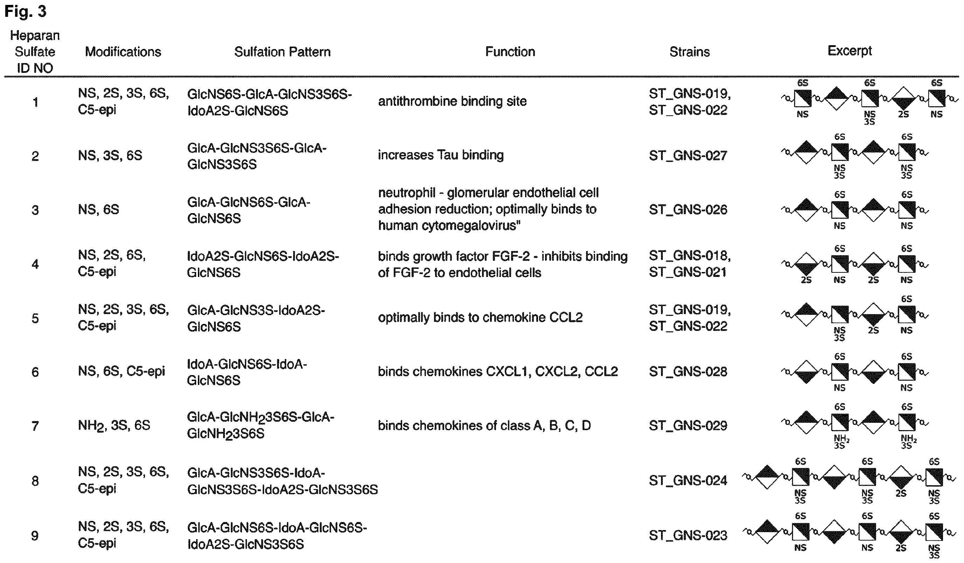

The large number of possible heparosan modifications, i.e. N-deacetylation, N-sulfation, C5-epimerization, 2-O-sulfation, 6-O-sulfation, 3-O-sulfation as well as the combination of the modifications, results in an abundance of different heparosan derivatives. The complexity of possible enzymatically catalysed modifications of heparosan and their combinations is further extended by the differing substrate preferences of enzyme isoforms. This is particularly true for HS3ST isoforms, whose dissimilar substrate preferences mediate various distinct sulfation patterns (SP). This amount of structural information is translated into a plethora of biological functions, the scope of which has yet to be clarified. Due to the usage of heparin as anticoagulant, the SP that causes antithrombin binding to the haparan sulfate (GlcNS6S-GlcA-GlcnS3S6s-IdoA2S-GlcNS6S) is well characterised. However, the biological functions of heparan sulfates with different SPs and their action mechanisms have been studied only recently. Initial research on the topic suggests that heparan sulfates with defined modifications and SPs mediate a broad range of biological functions, enabling their therapeutic applications as anti-cancer, anti-inflammatory, antimicrobial, anti-diabetic or anti-neurodegenerative drugs (Fig. 3). The research on biological functions and therapeutic application of structurally well-defined heparan sulfates has been hampered by the absence of production capabilities. To date, the large majority of heparan sulfate is sourced from animal tissue, with no control over the kind and extent of enzymatic modifications, leading to heterogeneous heparan sulfate. Cell factories, in particular yeast cell factories, provide a solution for industrial scale production of homogeneous, structurally well-defined heparan sulfates. This is because yeast cell factories allow for precise control over the kind and extent of enzymatic modifications of heparosan, since enzyme expression can be tightly controlled.

To obtain yeast cell factories that produce heparan sulfates with distinct modifications and sulfation patterns, the required heterologous genes for the heparosan modifications were stably integrated into the yeast genome. Since many of the required enzymes are natively localized in the endoplasmic reticulum and Golgi apparatus, the genes encoding the respective enzymes were truncated to exclude the N-terminal membrane domain (MD) or N-terminal signal peptide/ signal domain (SD). Thus, cytosolic localisation was achieved. To engineer a yeast strain (or a yeast cell, respectively) capable of industrial scale production of heparan sulfate containing the antithrombin binding SP (i.e. heparin), NDST1-ΔMD, C5-epimerase-ΔMD, HS2ST1-ΔMD, HS6ST1-ΔMD, and HS3ST1-ΔSD was integrated into the genome of the previously engineered ST_GNS-016. To achieve the correct SP specifically regarding 3-O-sulfation, the integration of HS3ST1, HS3ST3, HS3ST5 or a HS3ST1-like HS3ST-like, HS3ST5-like sulfate transferase is crucial. Heparan sulfate, characterised by N-, 3- and 6-O-sulfation has been shown to bind well to the protein Tau which is implicated in causing Alzheimer's disease. Therefore, N-, 3- and 6-O-sulfated heparan sulfate might be a viable therapeutic approach to combat the disease [5]. To engineer a yeast strain for the industrial scale production of N-, 3- and 6-O-sulfated heparan sulfate, NDST1-ΔMD, HS6ST1-ΔMD, and HS3ST5-ΔMD were stably integrated into the previously engineered strain ST_GNS-016. Similar to the well-defined heparan sulfates that mediate either antithrombin or Tau binding, a number of other modifications and SPs are known to mediate specific binding of cytokines, growth factors and other proteins. Yeast strains were engineered to produce heparan sulfates with these specific modifications and SPs (Fig. 3).

The present invention provides the following aspects, subject-matters and preferred embodiments, which respectively taken alone or in combination, contribute to solving the object of the present invention:
(1) Recombinant yeast cell, characterized in that it exhibits, preferably produces, an increased amount of UDP-GlcA (UDP-D-glucuronic acid) and/or UDP-GlcNAc (UDP-N-acetyl-D-glucosamine); and optionally PAPS (3'-phospho-adenylylsulfate), when compared to its wildtype cell.

The yeast cell can be any type of yeast cell. In a preferred embodiment, the yeast cell is of the genus selected from the group consisting of Saccharomyces, Pichia, Yarrowia, Kluyveromyces, Candida, Rhodotorula, Rhodosporidium, Cryptococcus, Trichosporon and Lipomyces, such as Saccharomyces cerevisiae, Saccharomyces boulardi, Pichia pastoris, Kluyveromyces marxianus, Cryptococcus albidus, Lipomyces lipofera, Lipomyces starkeyi, Rhodosporidium toruloides, Rhodotorula glutinis, Trichosporon pullulan and Yarrowia lipolytica. In a more preferred embodiment, the genus is Saccharomyces. In a particularly preferred embodiment, the yeast cell is Saccharomyces cerevisiae (S. cerevisiae).

In a preferred embodiment, the recombinant yeast cell exhibits an increased amount of UDP-GlcA (UDP-D-glucuronic acid) and UDP-GlcNAc (UDP-N-acetyl-D-glucosamine); and optionally PAPS (3'-phospho-adenylyl-sulfate), when compared to its wildtype cell (or wildtype strain, respectively).

In a further preferred embodiment, the recombinant yeast cell exhibits an increased amount of UDP-GlcA (UDP-D-glucuronic acid), UDP-GlcNAc (UDP-N-acetyl-D-glucosamine) and PAPS (3'-phospho-adenylyl-sulfate), when compared to its wildtype strain.

Whether or not the recombinant yeast cell exhibits or produces an increased amount of the respective matter (e.g. precursor) can e.g. be determined by HPLC analysis. When determining the amount of the expressed/produced matter of two cell strains, both strains are subjected to the same procedure in order to ensure comparability of the obtained results.

In a preferred embodiment, this increased amount of UDP-GlcA (UDP-D-glucuronic acid) and/or UDP-GlcNAc (UDP-N-acetyl-D-glucosamine); and optionally PAPS (3'-phospho-adenylyl-sulfate) is arrived at by modulating gene(s) that is(are) involved in the biosynthetic pathway of UDP-GlcA (UDP-D-glucuronic acid), UDP-GlcNAc (UDP-N-acetyl-D-glucosamine), and/or PAPS (3'-phospho-adenylyl-sulfate), depending on which gene(s) are desired to be modulated. The pathway is depicted in Fig. 1a. The modulation of genes can result in an overexpression, or a downregulation, of the gene. In general, overexpression results in an increased gene product, and downregulation results in a decreased gene product. Downregulation and overexpression of a certain gene can be arrived at by any suitable mean that is known to a person skilled in the art.
(2) Recombinant yeast cell according to item (1), characterized in that it exhibits, preferably expresses, an increased amount of one or more enzymes selected from the group consisting of: UTP-glucose-1-phosphat-uridylyltransferase (UDP-glucose pyrophosphorylase), phosphoglucomutase, glutamine-fructose-6-phosphate transaminase, N-acetyl-glucosamine-phosphate mutase, and optionally ATP-sulfurylase, adenylyl-sulfate kinase, and bisphosphate-3'-nucelotidase when compared to its wildtype strain.

In a preferred embodiment, the UDP-glucose pyrophosphorylase is encoded by a nucleotide (nt) sequence which comprises, preferably consists of, nucleotide (nt) sequence: SEQ ID NO: 30. The corresponding amino acid sequence (protein sequence) is: SEQ ID NO: 29.

In a preferred embodiment, the phosphoglucomutase is encoded by a nucleotide (nt) sequence which comprises, preferably consists of, nucleotide (nt) sequence: SEQ ID NO: 26 or SEQ ID NO: 28 respectively. The corresponding amino acid sequence (protein sequence) is: SEQ ID NO: 25 SEQ ID NO: 27 respectively.

In a preferred embodiment, the glutamine-fructose-6-phosphate transaminase is encoded by a nucleotide (nt) sequence which comprises, preferably consists of, nucleotide (nt) sequence: SEQ ID NO: 32. The corresponding amino acid sequence (protein sequence) is: SEQ ID NO: 31.

In a preferred embodiment, the N-acetyl-glucosamine-phosphate mutase is encoded by a nucleotide (nt) sequence which comprises, preferably consists of, nucleotide (nt) sequence: SEQ ID NO: 34. The corresponding amino acid sequence (protein sequence) is: SEQ ID NO: 33.

In a preferred embodiment, the ATP-sulfurylase is encoded by a nucleotide (nt) sequence which comprises, preferably consists of, nucleotide (nt) sequence: SEQ ID NO: 36. The corresponding amino acid sequence (protein sequence) is: SEQ ID NO: 35.

In a preferred embodiment, the adenylyl-sulfate kinase is encoded by a nucleotide (nt) sequence which comprises, preferably consists of, nucleotide (nt) sequence: SEQ ID NO: 38. The corresponding amino acid sequence (protein sequence) is: SEQ ID NO: 37.

In a preferred embodiment, the bisphosphate-3'-nucelotidase is encoded by a nucleotide (nt) sequence which comprises, preferably consists of, nucleotide (nt) sequence: SEQ ID NO: 40. The corresponding amino acid sequence (protein sequence) is: SEQ ID NO: 39.

In a preferred embodiment, the recombinant yeast cell expresses an increased amount of the enzymes UTP-glucose-1-phosphat-uridylyltransferase (UDP-glucose pyrophosphorylase), phosphoglucomutase, glutamine-fructose-6-phosphate transaminase, N-acetyl-glucosamine-phosphate mutase, ATP-sulfurylase, adenylyl-sulfate kinase, and bisphosphate-3'-nucelotidase, when compared to its wildtype strain.
(3) Recombinant yeast cell according to item (1), characterized in that
- (i) one or more of the genes that are involved in UDP-GlcA synthesis; and/or
   (ii) one or more of the genes that are involved in UDP-GlcNAc synthesis; and optionally
   (iii) one or more of the genes that are involved in PAPS synthesis are overexpressed; and

- a gene that encodes heterologous UDP-glucose dehydrogenase is expressed; thereby resulting in an increased amount of UDP-GlcA, UDP-GlcNAc, and optionally PAPS, when compared to the expression of the respective gene(s) in its wildtype strain.

In order to determine whether a defined gene is overexpressed compared to the expression of said gene in a wildtype cell, any suitable method that is known to a skilled person can be used. For example, mRNA-level can be determined, e.g. by transcriptional analysis as disclosed elsewhere herein.

In a preferred embodiment,
- (i) one or more of the genes that are involved in UDP-GlcA synthesis; and
   (ii) one or more of the genes that are involved in UDP-GlcNAc synthesis; and optionally
   (iii) one or more of the genes that are involved in PAPS synthesis are overexpressed; and
- a gene that encodes heterologous UDP-glucose dehydrogenase is expressed; thereby resulting in an increased amount of UDP-GlcA, UDP-GlcNAc, and optionally PAPS, when compared to its wildtype strain.

In a further preferred embodiment,
- (i) one or more of the genes that are involved in UDP-GlcA synthesis; and
   (ii) one or more of the genes that are involved in UDP-GlcNAc synthesis; and
   (iii) one or more of the genes that are involved in PAPS synthesis are overexpressed; and
- a gene that encodes heterologous UDP-glucose dehydrogenase is expressed; thereby resulting in an increased amount of UDP-GlcA, UDP-GlcNAc, and PAPS, when compared to its wildtype strain.
(4) Recombinant yeast cell of item (3), characterized in that
- the genes that are involved in (i) UDP-GlcA synthesis are genes that encode phosphoglucomutase, preferably phosphoglucomutase 1/2 (PGM1/2); and UTP-glucose-1-phosphat-uridylyltransferase (UDP-glucose pyrophosphorylase), preferably UDP-glucose pyrophosphorylase 1 (UGP1);
- the genes that are involved in (ii) UDP-GlcNAc synthesis are genes that encode glutamine-fructose-6-phosphate transaminase, preferably Glutamine-fructose-6-phosphate amidotransferase 1 (GFA1); and N-acetyl-glucosamine-phosphate mutase, preferably Phosphoacetylglucosamine Mutase 1 (PCM1); and/or
- the genes that are involved in (iii) PAPS synthesis are genes that encode sulfate adenylyltransferase (ATP-sulfurylase), preferably METhionine requiring protein 3 (MET3); adenylyl-sulfate kinase, preferably METhionine requiring protein 14 (MET14); and bisphosphate-3'-nucleotidase, preferably METhionine requiring protein 22 (MET22).

In a preferred embodiment, the phosphoglucomutase 1/2 (PGM1/2) is encoded by a nucleotide (nt) sequence which comprises, preferably consists of, nucleotide (nt) sequence: SEQ ID NO: 26 or SEQ ID NO: 28 respectively. The corresponding amino acid sequence (protein sequence) is: SEQ ID NO: 25 or SEQ ID NO: 27 respectively.

In a preferred embodiment, the UDP-glucose pyrophosphorylase 1 (UGP1) is encoded by a nucleotide (nt) sequence which comprises, preferably consists of, nucleotide (nt) sequence: SEQ ID NO: 30. The corresponding amino acid sequence (protein sequence) is: SEQ ID NO: 29.

In a preferred embodiment, the glutamine-fructose-6-phosphate transaminase 1 (GFA1) is encoded by a nucleotide (nt) sequence which comprises, preferably consists of, nucleotide (nt) sequence: SEQ ID NO: 32. The corresponding amino acid sequence (protein sequence) is: SEQ ID NO: 31.

In a preferred embodiment, the phosphoacetylglucosamine Mutase 1 (PCM1) is encoded by a nucleotide (nt) sequence which comprises, preferably consists of, nucleotide (nt) sequence: SEQ ID NO: 34. The corresponding amino acid sequence (protein sequence) is: SEQ ID NO: 33.

In a preferred embodiment, the METhionine requiring protein 3 (MET3) is encoded by a nucleotide (nt) sequence which comprises, preferably consists of, nucleotide (nt) sequence: SEQ ID NO: 36. The corresponding amino acid sequence (protein sequence) is: SEQ ID NO: 35.

In a preferred embodiment, the METhionine requiring protein 14 (MET14) is encoded by a nucleotide (nt) sequence which comprises, preferably consists of, nucleotide (nt) sequence: SEQ ID NO: 38. The corresponding amino acid sequence (protein sequence) is: SEQ ID NO: 37.

In a preferred embodiment, the METhionine requiring protein 22 (MET22) is encoded by a nucleotide (nt) sequence which comprises, preferably consists of, nucleotide (nt) sequence: SEQ ID NO: 40. The corresponding amino acid sequence (protein sequence) is: SEQ ID NO: 39.

In a preferred embodiment, the overexpression of phosphoglucomutase (PGM) can be achieved by chromosomal integration of an additional gene copy of encoding the phosphoglucomutase (PGM) together with a strong promoter pTEF1 upstream of said gene.

In a preferred embodiment, the overexpression of UDP-glucose pyrophosphorylase (UGP) can be achieved by chromosomal integration of an additional gene copy encoding the UTP-glucose-1-phosphate-uridylyltransferase (UGP) together with a strong promoter pTDH3 upstream of said gene.

In a preferred embodiment, the overexpression of glutamine-fructose-6-phosphate transaminase (GFA) can be achieved by chromosomal integration of an additional gene copy encoding the UTP-glucose-1-phosphate-uridylyltransferase (GFA) together with a strong promoter pTEF1 upstream of said gene.

In a preferred embodiment, the overexpression of phosphoacetylglucosamine Mutase (PCM) can be achieved by chromosomal integration of an additional gene copy encoding the UTP-glucose-1-phosphate-uridylyltransferase (PCM) together with a strong promoter pTDH3 upstream of said gene.

In a preferred embodiment, the overexpression of METhionine requiring protein 3 (MET3) can be achieved by chromosomal integration of an additional gene copy of MET3 together with a strong promoter pTDH3 upstream of said gene.

In a preferred embodiment, the overexpression of METhionine requiring protein 14 (MET14) can be achieved by chromosomal integration of an additional gene copy of MET14 together with a strong promoter pTEF1 upstream of said gene.

In a preferred embodiment, the overexpression of METhionine requiring protein 22 (MET22) can be achieved by chromosomal integration of an additional gene copy of MET22 together with a strong promoter pPGK1 upstream of said gene.
(5) Recombinant yeast cell of any of the preceding items, characterized in that
- a gene encoding the enzyme chitin-synthase, preferably chitin-synthase 2/3 (CHS2/3), is downregulated, to the effect that the amount of the chitin-synthase is decreased; and/or
- a gene encoding the enzyme 3'-phosphoadenylylsulfate reductase, preferably MET16 is downregulated,
to the effect that the amount of the respective enzyme the down-regulated gene encodes is decreased, when compared to its wildtype strain.

In a preferred embodiment, the chitin-synthase 2/3 (CHS2/3) is encoded by a nucleotide (nt) sequence which comprises, preferably consists of, nucleotide (nt) sequence: SEQ ID NO: 42 or SEQ ID NO: 44 respectively. The corresponding amino acid sequence (protein sequence) is: SEQ ID NO: 41 or SEQ ID NO: 43 respectively.

In a preferred embodiment, the 3'-phosphoadenylyl-sulfate reductase (MET16) is encoded by a nucleotide (nt) sequence which comprises, preferably consists of, nucleotide (nt) sequence: SEQ ID NO: 46. The corresponding amino acid sequence (protein sequence) is: SEQ ID NO: 45.
(6) Recombinant yeast cell of item (5), characterized in that the downregulation of the respective gene is effected by decreasing the transcription of said gene.
(7) Recombinant yeast cell of item (6), characterized in that decreasing the transcription is effected by replacing the native promoter of the gene to be downregulated by a comparably weaker promoter.
(8) Recombinant yeast cell of any one of item (6) or (7), characterized in that decreasing the transcription is effected by replacing the native promoter of CHS2/3 by KEX2 promoter (pKEX2), and/or the native promoter of MET16 by KEX2 promoter (pKEX2).

In a preferred embodiment, the native promoter of MET16 is encoded by a nucleotide (nt) sequence which comprises, preferably consists of, nucleotide (nt) sequence: SEQ ID NO: 48.

In a preferred embodiment, the native CHS3 promoter is encoded by a nucleotide (nt) sequence which comprises, preferably consists of, nucleotide (nt) sequence: SEQ ID NO: 47.

In a preferred embodiment, the KEX2 promoter is encoded by a nucleotide (nt) sequence which comprises, preferably consists of, nucleotide (nt) sequence: SEQ ID NO: 49.

The present invention also refers to a recombinant yeast cell according to any of the preceding items, characterized in that it additionally expresses gene(s) encoding a UDP-glucose dehydrogenase, thereby being able to produce UDP-GlcA (UDP-D-glucuronic acid).

In a preferred embodiment, the UDP-glucose dehydrogenases is encoded by a nucleotide (nt) sequence which comprises, preferably consists of, nucleotide (nt) sequence: SEQ ID NO: 1. The corresponding amino acid sequence (protein sequence) is: SEQ ID NO: 2.
(9) Recombinant yeast cell according to any of the preceding items, characterized in that it exhibits an increased amount of UDP-GlcA (UDP-D-glucuronic acid), UDP-GlcNAc (UDP-N-acetyl-D-glucosamine), and PAPS (3'-phospho-adenylylsulfate), when compared to its wildtype strain.
(10) Recombinant yeast cell according to any of the preceding items, characterized in that it additionally comprise gene(s) encoding a glycosaminoglycan synthase, thereby being able to produce glycosaminoglycans such as heparosan, hyaluronic acid, dermatan, chondroitin or their sulfated analogs; preferably said recombinant yeast cell additionally expresses gene(s) encoding a glycosaminoglycan synthase, thereby being able to produce glycosaminoglycans such as heparosan, hyaluronic acid, dermatan, chondroitin or their sulfated analogs.
(11) Recombinant yeast cell according to any of the preceding items, characterized in that it additionally comprises gene(s) encoding a heparosan synthase, preferably heparosan synthase 1 and/or heparosan synthase 2, thereby being able to produce heparosan; preferably said recombinant yeast cell additionally expresses gene(s) encoding a heparosan synthase, preferably heparosan synthase 1 and/or heparosan synthase 2, thereby producing heparosan.

The recombinant yeast cell of (10) and (11), by expressing gene(s) encoding a glycosaminoglycan synthase and a heparosan synthase, exhibits, preferably expresses, preferably an increased amount of glycosaminoglycan synthase and heparosan synthase, when compared to its wildtype strain.

The gene(s) encoding glucosaminoglycan synthase and heparosan synthase, respectively, are heterologously expressed in the recombinant yeast cell that is capable of UDP-GlcA and UDP-GlcNAc biosynthesis. For this purpose, the respective genes are integrated into the genome of the recombinant yeast cell according to item (1) to (9) by applying suitable techniques that are known to a skilled person, as described elsewhere herein.
(12) Recombinant yeast cell according to item (11), characterized in that the heparosan synthase is heparosan synthase 1 (PmHS1) or a like-form of PmHS1, and/or Heparosan synthase 2 (PmHS2) or a like-form of PmHS2, preferably PmHS1 and PmHS2 are expressed.

In a preferred embodiment, the Heparosan synthase 1 (PmHS1) is encoded by a nucleotide (nt) sequence which comprises, preferably consists of, nucleotide (nt) sequence: SEQ ID NO: 4. The corresponding amino acid sequence (protein sequence) is: SEQ ID NO: 3.

In a preferred embodiment, the Heparosan synthase 2 (PmHS2) is encoded by a nucleotide (nt) sequence which comprises, preferably consists of, nucleotide (nt) sequence: SEQ ID NO: 6. The corresponding amino acid sequence (protein sequence) is: SEQ ID NO: 5.
(13) Recombinant yeast cell strain according to item (12), characterized in that heparosan synthase PmHS1 and/or PmHS2 is fused with a nuclear export sequence (NES), preferably with an NES being encoded by a nucleotide (nt) sequence comprising, or preferably consisting of, SEQ ID NO: 54, of S. cerevisiae's Hsp70-type chaperone SSB1, to the C-termini of the heparosan synthases, thereby being capable of expressing heparosan synthase PmHS1 and/or PmHS2 specifically in cytosol, rather than localizing in the yeast nucleus, thereby increasing the production of heparosan.

The amino acid sequence corresponding to SEQ ID NO: 54 is SEQ ID NO: 53.

In a preferred embodiment, the amino acid sequence of Hsp70-type chaperone SSB1 comprises, preferably consists of, SEQ ID NO: 56, encoded by a nucleotide (nt) sequence comprising, or preferably consisting of, SEQ ID NO: 55.
(14) A method of producing heparosan using a recombinant yeast cell of item (11) or (13), preferably the S. cerevisiae strain is ST_GNS_016.
(15) Recombinant yeast cell according to any one of items (11) to (13), or as defined in item (14), characterized in that it additionally comprises gene(s) encoding N-deacetylase (ND), and/or N-deacetylase-N-sulfotransferase (NDST), and/or C5-epimerase (C5-epi), and/or 2-O-sulfotransferase (HS2ST), and/or 3-O-sulfotransferase (HS3ST), and/or 6-O-sulfotransferase (HS6ST), thereby being capable of producing heparan sulfates; preferably said recombinant yeast cell additionally expresses gene(s) encoding N-deacetylase (ND), and/or N-deacetylase-N-sulfotransferase (NDST), and/or C5-epimerase (C5-epi), and/or 2-O-sulfotransferase (HS2ST), and/or 3-O-sulfotransferase (HS3ST), and/or 6-O-sulfotransferase (HS6ST), thereby being capable of producing heparan sulfates.

In a preferred embodiment, the heparan sulfate that is produced by the recombinant S. cerevisiae strain is heparin.
(16) Recombinant yeast cell according to any one of items (11) to (13), characterized in that it additionally comprises gene(s) encoding ND, and/or, NDST, and/or C5-epi, and/or HS2ST, and/or HS3ST, and/or HS6ST truncated to exclude the N-terminal membrane domain (MD) or N-terminal signal peptide/ signal domain (SD); preferably said recombinant yeast cell expresses gene(s) encoding ND, and/or, NDST, and/or C5-epi, and/or HS2ST, and/or HS3ST, and/or HS6ST truncated to exclude the N-terminal membrane domain (MD) or N-terminal signal peptide/ signal domain (SD), thereby expressing ND, and/or NDST, and/or C5-epi, and/or HS2ST, and/or HS3ST, and/or HS6ST enzymes specifically in cytosol, rather than localizing in the yeast endoplasmic reticulum, thereby increasing the production of heparan sulfates with heterogeneously well-defined sulfation patterns and/or heparin.
(17) Recombinant yeast cell according to item (15) or (16), characterized in that the N-deacetylase is ND1 and/or ND1-like; N-sulfotransferase is NDST1 and/or NDST1-like; 2-O-sulfotransferase is HS2ST1 and/or HS2ST1-like; the 3-O-sulfotransferase is HS3ST1, HS3ST3, or HS3ST5, and/or HS3ST1-like, HS3ST3-like, or HS3ST5-like respectively; and 6-O-sulfotransferase is HS6ST1 and/or HS6ST1-like.
(18) Recombinant yeast cell according to item (15), characterized in that genes encoding NDST, C5-epi, HS2ST, HS3ST1 and/or HS3ST1-like, and HS6ST are additionally expressed, thereby being able to produce heparan sulfate as depicted in Formula (1) in Fig. 3.
(19) Recombinant yeast cell according to item (15), characterized in that genes encoding NDST, HS3ST5 and/or HS3ST5, and HS6ST are additionally expressed, thereby being able to produce heparan sulfates as depicted in Formula (2) in Fig. 3.
(20) Recombinant yeast cell according to item (15), characterized in that genes encoding NDST, and HS6ST are additionally expressed, thereby being able to produce heparan sulfates as depicted in Formula (3) in Fig. 3.
(21) Recombinant yeast cell according to item (15), characterized in that genes encoding NDST, C5-epi, HS2ST, and HS6ST are additionally expressed, thereby being able to produce heparan sulfates as depicted in Formula (4) in Fig. 3.
(22) Recombinant yeast cell according to item (15), characterized in that genes encoding NDST, C5-epi, HS2ST, HS3ST1 and/or HS3ST1-like, and HS6ST are additionally expressed, thereby being able to produce heparan sulfate as depicted in Formula (5) in Fig. 3.
(23) Recombinant yeast cell according to item (15), characterized in that genes encoding NDST, C5-epi, and HS6ST are additionally expressed, thereby being able to produce heparan sulfate as depicted in Formula (6) in Fig. 3.
(24) Recombinant yeast cell according to item (15), characterized in that genes encoding ND, HS3ST5 and/or HS3ST5-like, and HS6ST are additionally expressed, thereby being able to produce heparan sulfate as depicted in Formula (7) in Fig. 3.
(25) Recombinant yeast cell according to item (15), characterized in that genes encoding NDST, C5-epi, HS2ST, HS3ST5 and/or HS3ST5-like, and HS6ST are additionally expressed, thereby being able to produce heparan sulfate as depicted in Formula (8) in Fig. 3.
(26) Recombinant yeast cell according to item (15), characterized in that genes encoding NDST, C5-epi, HS2ST, HS3ST3 and/or HS3ST3-like, and HS6ST are additionally expressed, thereby being able to produce heparan sulfate as depicted in Formula (9) in Fig. 3.
(27) Recombinant yeast cell according to item (16), characterized in that genes encoding NDST-ΔMD, C5-epi-ΔMD, HS2ST-ΔMD, HS3ST1-ΔSD and/or HS3ST1-ΔSD-like, and HS6ST-ΔMD are additionally expressed, thereby being able to produce increased cytosolic concentration of heparan sulfate as depicted in Formula (1) in Fig. 3.

In a preferred embodiment, the recombinant yeast cell is S. cerevisiae strain ST_GNS-019, or ST_GNS-022.
(28) Recombinant yeast cell according to item (16), characterized in that genes encoding NDST-ΔMD, HS3ST5-ΔMD and/or HS3ST5-ΔMD, and HS6ST-ΔMD are additionally expressed, thereby being able to produce increased cytosolic concentration of heparan sulfates as depicted in Formula (2) in Fig. 3.

In a preferred embodiment, the recombinant yeast cell is S. cerevisiae strain ST_GNS-027.
(29) Recombinant yeast cell according to item (16), characterized in that genes encoding NDST-ΔMD, and HS6ST-ΔMD are additionally expressed, thereby being able to produce increased cytosolic concentration of heparan sulfates as depicted in Formula (3) in Fig. 3.

In a preferred embodiment, the recombinant yeast cell is S. cerevisiae strain ST_GNS-026.
(30) Recombinant yeast cell according to item (16), characterized in that genes encoding NDST-ΔMD, C5-epi-ΔMD, HS2ST-ΔMD, and HS6ST-ΔMD are additionally expressed, thereby being able to produce increased cytosolic concentration of heparan sulfates as depicted in Formula (4) in Fig. 3.

In a preferred embodiment, the recombinant yeast cell is S. cerevisiae strain ST_GNS-018, or ST_GNS-021.
(31) Recombinant yeast cell according to item (16), characterized in that genes encoding NDST-ΔMD, C5-epi-ΔMD, HS2ST-ΔMD, HS3ST1-ΔSD and/or HS3ST1-ΔSD, and HS6ST-ΔMD are additionally expressed, thereby being able to produce increased cytosolic concentration of heparan sulfate as depicted in Formula (5) in Fig. 3.

In a preferred embodiment, the recombinant yeast cell is S. cerevisiae strain ST_GNS-019, or ST_GNS-022.
(32) Recombinant yeast cell according to item (16), characterized in that genes encoding NDST-ΔMD, C5-epi-ΔMD, and HS6ST-ΔMD are additionally expressed, thereby being able to produce increased cytosolic concentration of heparan sulfate as depicted in Formula (6).

In a preferred embodiment, the yeast cell is recombinant S. cerevisiae strain ST_GNS-028.
(33) Recombinant yeast cell according to item (16), characterized in that genes encoding ND-ΔMD, HS3ST5-ΔMD and/or HS3ST5-ΔMD, and HS6ST-ΔMD are additionally expressed, thereby being able to produce increased cytosolic concentration of heparan sulfate as depicted in Formula (7)

In a preferred embodiment, the recombinant yeast cell is S. cerevisiae strain ST_GNS-027.
(34) Recombinant yeast cell according to item (16), characterized in that genes encoding NDST-ΔMD, C5-epi-ΔMD, HS2ST-ΔMD, HS3ST5-ΔMD and/or HS3ST5-ΔMD, and HS6ST-ΔMD are additionally expressed, thereby being able to produce increased cytosolic concentration of heparan sulfate as depicted in Formula (8) in Fig. 3.

In a preferred embodiment, the recombinant yeast cell is S. cerevisiae strain ST_GNS-024.
(35) Recombinant yeast cell according to item (16), characterized in that genes encoding NDST-ΔMD, C5-epi-ΔMD, HS2ST-ΔMD, HS3ST3-ΔMD and/or HS3ST3-ΔMD, and HS6ST-ΔMD are additionally expressed, thereby being able to produce increased cytosolic concentration of heparan sulfate as depicted in Formula (9) in Fig. 3.

In a preferred embodiment, the recombinant yeast cell is S. cerevisiae strain ST_GNS-023.
(36) Recombinant S. cerevisiae strains ST_GNS-001, ST_GNS-002, ST_GNS-003, ST_GNS-004, ST_GNS-005, ST_GNS-006, ST_GNS-007, ST_GNS-008, ST_GNS-009, ST_GNS-010, ST_GNS-011, ST_GNS-012, ST_GNS-013, ST_GNS-014, ST_GNS-015, ST_GNS-016, ST_GNS-017, ST_GNS-018, ST_GNS-019, ST_GNS-020, ST_GNS-021, ST_GNS-022, ST_GNS-023, ST_GNS-024, ST_GNS-025, ST_GNS-026, ST_GNS-027, ST_GNS-028, and ST_GNS-029, respectively.

ST_GNS-014 is deposited at the DSMZ (German Collection of Microorganisms and Cell Cultures GmbH) with the deposition number DSM 34100.

ST_GNS-022 is deposited at the DSMZ with the deposition number DSM 34101.
(37) Recombinant yeast cell according to any one of items (18) to (35) or recombinant S. cerevisiae strain according to item (36), characterized in that it produces the heparan sulfates comprise a length of 4 or more saccharide residues.
(38) A recombinant microorganism, capable of heparan sulfate production.
(39) A method of producing heparan sulfates using a recombinant microorganism, preferably the recombinant microorganism of the present invention.

In order to produce the heparan sulfates, the production strains (that is, the recombinant microorganisms or recombinant yeast strains, respectively), are cultivated under suitable conditions in a cultivation broth. The cultivation of the respective strains (production strains), as well as the purification of the product, is carried out according to routine protocols that are well known to a person of skill in the art.

For example, the production strains are grown in suitable cultivation containers such as flasks or vessels for a suitable, defined time period, e.g. 28 hours to 144 hours, preferably 48 hours to 96 hours, in a suitable cultivation broth. The cultures are centrifuged, and the recovered supernatant is filtered. The filtered supernatant is then purified e.g. by using a column: The supernatant is loaded onto the column and washed, followed by eluting the product from the column using suitable buffer. Then, the product is precipitated, followed by washing the pellet, thereby obtaining the purified product.

The thus-obtained purified product is then analyzed e.g. with regard to the sulfation pattern, by LC-MS analysis.
(40) A method of producing heparan sulfates using a recombinant yeast cell or recombinant S. cerevisiae strain according to any one of items (18) to (37).
(41) The heparan sulfates as depicted in Formula (1) to (9) in Fig. 3, preferably the heparan sulfates as depicted in Formulas (2) to (9) in Fig. 3.
(42) The heparan sulfates as depicted in Formula (1) in Fig. 3 obtained by cultivating (fermenting) the recombinant yeast strain(s) as defined in item (18) or (27).

The obtained heparan sulfates contain one or more sequences of residues as depicted in formula (1) (heparin). Thus, in a preferred embodiment, the present invention also refers to a heparan sulfate containing one or more sequences of residues as depicted in formula (1) (heparin) of Fig. 3 which is capable of binding antithrombine.
(43) The heparan sulfates as depicted in Formula (2) in Fig. 3 obtained by cultivating (fermenting) the recombinant yeast strain(s) as defined in item (19) or (28).

The obtained heparan sulfates contain one or more sequences of residues as depicted in formula (2). Thus, in a preferred embodiment, the present invention also refers to a heparan sulfate containing one or more sequences of residues as depicted in formula (2) of Fig. 3 which is capable of increasing Tau binding.
(44) The heparan sulfates as depicted in Formula (3) in Fig. 3 obtained by cultivating (fermenting) the recombinant yeast strain(s) as defined in item (20) or (29).

The obtained heparan sulfates contain one or more sequences of residues as depicted in formula (3). Thus, in a preferred embodiment, the present invention also refers to a heparan sulfate containing one or more sequences of residues as depicted in formula (3) of Fig. 3 which is capable of optimally binding human cytomegalovirus.
(45) The heparan sulfates as depicted in Formula (4) in Fig. 3 obtained by cultivating (fermenting) the recombinant yeast strains as defined in item (21) or (30).

The obtained heparan sulfates contain one or more sequences of residues as depicted in formula (4). Thus, in a preferred embodiment, the present invention also refers to a heparan sulfate containing one or more sequences of residues as depicted in formula (4) of Fig. 3, which is capable of binding growth factor FGF-2 thereby inhibiting the binding of FGF-2 to endothelial cells.
(46) The heparan sulfates as depicted in Formula (5) in Fig. 3 obtained by cultivating (fermenting) the recombinant yeast strains as defined in item (22) or (31).

The obtained heparan sulfates contain one or more sequences of residues as depicted in formula (5). Thus, in a preferred embodiment, the present invention also refers to a heparan sulfate containing one or more sequences of residues as depicted in formula (5) of Fig. 3, which is capable of optimally binding to chemokine CCL2.
(47) The heparan sulfates as depicted in Formula (6) in Fig. 3 obtained by cultivating (fermenting) the recombinant yeast strains as defined in item (23) or (32).

The obtained heparan sulfates contain one or more sequences of residues as depicted in formula (6). Thus, in a preferred embodiment, the present invention also refers to a heparan sulfate containing one or more sequences of residues as depicted in formula (6) of Fig. 3, which is capable of binding chemokines CXCL1, CXCL2, and CCL2.
(48) The heparan sulfates as depicted in Formula (7) in Fig. 3 obtained by cultivating (fermenting) the recombinant yeast strains as defined in item (24) or (33).

The obtained heparan sulfates contain one or more sequences of residues as depicted in formula (7). Thus, in a preferred embodiment, the present invention also refers to a heparan sulfate containing one or more sequences of residues as depicted in formula (7) of Fig. 3, which is capable of binding chemokines of class A, B, C, and D.
(49) The heparan sulfates as depicted in Formula (8) in Fig. 3 obtained by cultivating (fermenting) the recombinant yeast strains as defined in item (25) or (34).

The obtained heparan sulfates contain one or more sequences of residues as depicted in formula (8). Thus, in a preferred embodiment, the present invention also refers to a heparan sulfate containing one or more sequences of residues as depicted in formula (8) of Fig. 3.
(50) The heparan sulfates as depicted in Formula (9) in Fig. 3 obtained by cultivating (fermenting) of the recombinant yeast strains as defined in item (26) or (35).

The obtained heparan sulfates contain one or more sequences of residues as depicted in formula (9). Thus, in a preferred embodiment, the present invention also refers to a heparan sulfate containing one or more sequences of residues as depicted in formula (9) of Fig. 3.
(51) The heparan sulfates according to any one of the items (41) to (50), wherein the heparan sulfates comprise a length of 4 or more saccharide residues.
(52) Use of a recombinant yeast cell as defined in any one of items (1) to (37) in a method of producing heparan sulfate.
(53) Use of a recombinant microorganism as defined in item (38) in a method of producing heparan sulfate.
(54) Recombinant yeast cell of any of the preceding items, characterized in that the expression of heterologous genes and/or overexpression of endogenous genes is achieved by stable gene integration.

In a preferred embodiment, the stable integration does not rely on selection for resistance markers. Therefore, the resulting strains are marker-free.

### Definitions of terms as used within the meaning of the present invention

Within the meaning of the present invention, the term "cell strain" denotes cells derived from a single cell that possesses one or more specific features. Thus, a "cell strain" comprises multiple cells all possessing the same specific feature or specific features.

Within the meaning of the present invention, the term "gene" denotes a certain nucleic acid sequence that encodes a polypeptide or an RNA chain that has a function in the organism. As used herein, "nucleic acid" sequence includes DNA (desoxyribonucleic acid) or RNA (ribonucleic acid), in single stranded or double stranded form or otherwise.

In general, within the meaning of the present invention, the term "AtUGD1 gene" denotes a gene encoding UDP-glucose dehydrogenase 1 comprising (preferably consisting of) the nucleic acid sequence as depicted in the sequence depicted in SEQ ID NO: 2; the term "PmHS1 gene" denotes a gene encoding heparosan synthase 1 comprising (preferably consisting of) the nucleic acid sequence as depicted in the sequence depicted in SEQ ID NO: 4; the term "PmHS2 gene" denotes a gene encoding heparosan synthase 2 comprising (preferably consisting of) the nucleic acid sequence as depicted in the sequence depicted in SEQ ID NO: 6; the term "PmHS1_NES gene" denotes a gene encoding heparosan synthase 1 fused with a nuclear export sequence (NES) of S. cerevisiae's Hsp70-type chaperone SSB1 to the C-termini of the heparosan synthases comprising (preferably consisting of) the nucleic acid sequence as depicted in the sequence depicted in SEQ ID NO: 8; the term "PmHS2_NES gene" denotes a gene encoding heparosan synthase 2 fused with a nuclear export sequence (NES) of S. cerevisiae's Hsp70-type chaperone SSB1 to the C-termini of the heparosan synthases comprising (preferably consisting of) the nucleic acid sequence as depicted in the sequence depicted in SEQ ID NO: 10; As used herein , the term "-NES" denotes nuclear export signal, namely a short target peptide in a protein that targets said protein for export from the cell nucleus to the cytosol through the nuclear pore complex using nuclear transport. In a preferred embodiment, NES is encoded by a nucleotide (nt) sequence comprising, or preferably consisting of, SEQ ID NO: 54. The corresponding amino acid sequence (protein sequence) is: SEQ ID NO: 53. In another preferred embodiment, the amino acid sequence of SSB1 comprises, preferably consists of, SEQ ID NO: 56, encoded by a nucleotide (nt) sequence comprising, or preferably consisting of, SEQ ID NO: 55.

The term "ND1-ΔMD gene" denotes a gene encoding N-deacetylase 1 without encoding the N-terminal membrane domain comprising (preferably consisting of) the nucleic acid sequence as depicted in the sequence depicted in SEQ ID NO: 58; the term "NDST1-ΔMD gene" denotes a gene encoding N-deacetylase-N-sulfotransferase 1 without encoding the N-terminal membrane domain comprising (preferably consisting of) the nucleic acid sequence as depicted in the sequence depicted in SEQ ID NO: 12; the term "C5-epi-ΔMD gene" denotes a gene encoding D-glucuronyl C5-epimerase without encoding N-terminal membrane domain comprising (preferably consisting of) the nucleic acid sequence as depicted in the sequence depicted in SEQ ID NO: 14; the term "HS2ST1-ΔMD gene" denotes a gene encoding heparan sulfate 2-O-sulfotransferase 1 without encoding N-terminal membrane domain comprising (preferably consisting of) the nucleic acid sequence as depicted in the sequence depicted in SEQ ID NO: 16; the term "HS3ST1-ΔSD gene" denotes a gene encoding heparan sulfate 3-O-sulfotransferase 1 without encoding N-terminal signal domain (/N-terminal signal peptide) comprising (preferably consisting of) the nucleic acid sequence as depicted in the sequence depicted in SEQ ID NO: 18; the term "HS3ST3 A1-ΔMD gene" denotes a gene encoding heparan sulfate 3-O-sulfotransferase 3 A1 without encoding N-terminal membrane domain comprising (preferably consisting of) the nucleic acid sequence as depicted in the sequence depicted in SEQ ID NO: 20; the term "HS3ST5-ΔMD gene" denotes a gene encoding heparan sulfate 3-O-sulfotransferase 5 without encoding N-terminal membrane domain comprising (preferably consisting of) the nucleic acid sequence as depicted in the sequence depicted in SEQ ID NO: 22; and the term "HS6ST1-ΔMD gene" denotes a gene encoding heparan sulfate 6-O-sulfotransferase 1 without encoding N-terminal membrane domain comprising (preferably consisting of) the nucleic acid sequence as depicted in the sequence depicted in SEQ ID NO: 24. As used herein, the term "-ΔMD" denotes elimination of the N-terminal region of a gene that encodes the N-terminal membrane domain; the term "-ΔSD" denotes elimination of the N-terminal region of a gene that encodes the N-terminal signal peptide. Within the meaning of the present application, the term "domain" denotes a specific physical region or amino acid sequence in a protein which is associated with a particular function or corresponding segment of DNA. Within the meaning of the invention, the term "N-terminal membrane domain" denotes the first domain of the protein that is located at the N-terminus of the protein and inserts into or otherwise attaches to lipid bilayers. Within the meaning of the invention, the term "N-terminal signal peptide" denotes a peptide sequence located at the N-terminus of the protein that directs the transport of the protein to a specific cell organelle.

The term "PGM1 gene" denotes a gene encoding phosphoglucomutase 1 comprising (preferably consisting of) the nucleic acid sequence as depicted in the sequence depicted in SEQ ID NO: 26; the term "PGM2 gene" denotes a gene encoding phosphoglucomutase 2 comprising (preferably consisting of) the nucleic acid sequence as depicted in the sequence depicted in SEQ ID NO: 28; the term "UGP1 gene" denotes a gene encoding UDP-glucose pyrophosphorylase 1 comprising (preferably consisting of) the nucleic acid sequence as depicted in the sequence depicted in SEQ ID NO: 30; the term "GFA1 gene" denotes a gene encoding glutamine-fructose-6-phosphate amidotransferase 1 comprising (preferably consisting of) the nucleic acid sequence as depicted in the sequence depicted in SEQ ID NO: 32; the term "PCM1 gene" denotes a gene encoding phosphoacetylglucosamine mutase 1 comprising (preferably consisting of) the nucleic acid sequence as depicted in the sequence depicted in SEQ ID NO: 34; the term "MET3 gene" denotes a gene encoding methionine requiring protein 3 comprising (preferably consisting of) the nucleic acid sequence as depicted in the sequence depicted in SEQ ID NO: 36; the term "MET14 gene" denotes a gene encoding methionine requiring protein 14 comprising (preferably consisting of) the nucleic acid sequence as depicted in the sequence depicted in SEQ ID NO: 38; the term "MET22 gene" denotes a gene encoding methionine requiring protein 22 comprising (preferably consisting of) the nucleic acid sequence as depicted in the sequence depicted in SEQ ID NO: 40; the term "CHS2 gene" denotes a gene encoding chitin-synthase 2 comprising (preferably consisting of) the nucleic acid sequence as depicted in the sequence depicted in SEQ ID NO: 42; the term "CHS3 gene" denotes a gene encoding chitin-synthase 3 comprising (preferably consisting of) the nucleic acid sequence as depicted in the sequence depicted in SEQ ID NO: 44; the term "MET16 gene" denotes a gene encoding 3'-phosphoadenylyl-sulfate reductase comprising (preferably consisting of) the nucleic acid sequence as depicted in the sequence depicted in SEQ ID NO: 46; the term "gRNA (helper gRNA vector) carrying a natMX6 nourseothricin resistance marker" denotes an artificial synthetic nucleotide sequence as depicted in the sequence depicted in SEQ ID NO: 59.

Within the meaning of the present invention, the term "UDP-glucose dehydrogenase 1" denotes the protein comprising (preferably consisting of) the amino acid sequence as depicted in the sequence depicted in SEQ ID NO: 1; the term "heparosan synthase 1" denotes the protein comprising (preferably consisting of) the amino acid sequence as depicted in the sequence depicted in SEQ ID NO: 3; the term "heparosan synthase 2" denotes the protein comprising (preferably consisting of) the amino acid sequence as depicted in the sequence depicted in SEQ ID NO: 5; the term "heparosan synthase 1 encoded by PmHS1_NES gene" denotes the protein comprising (preferably consisting of) the amino acid sequence as depicted in the sequence depicted in SEQ ID NO: 7; the term "heparosan synthase 2 encoded by PmHS2_NES gene" denotes the protein comprising (preferably consisting of) the amino acid sequence as depicted in the sequence depicted in SEQ ID NO: 9; the term "N-deacetylase 1 without encoding N-terminal membrane domain " denotes the protein comprising (preferably consisting of) the amino acid sequence as depicted in the sequence depicted in SEQ ID NO: 57; the term "N-deacetylase-N-sulfotransferase 1 without encoding N-terminal membrane domain" denotes the protein comprising (preferably consisting of) the amino acid sequence as depicted in the sequence depicted in SEQ ID NO: 11; the term "D-glucuronyl C5-epimerase without encoding N-terminal membrane domain" denotes the protein comprising (preferably consisting of) the amino acid sequence as depicted in the sequence depicted in SEQ ID NO: 13; the term "heparan sulfate 2-O-sulfotransferase 1 without encoding N-terminal membrane domain" denotes the protein comprising (preferably consisting of) the amino acid sequence as depicted in the sequence depicted in SEQ ID NO: 15; the term "heparan sulfate 3-O-sulfotransferase 1 without encoding N-terminal signal domain" denotes the protein comprising (preferably consisting of) the amino acid sequence as depicted in the sequence depicted in SEQ ID NO: 17; the term "heparan sulfate 3-O-sulfotransferase 3 A1 without encoding N-terminal membrane domain" denotes the protein comprising (preferably consisting of) the amino acid sequence as depicted in the sequence depicted in SEQ ID NO: 19; the term "heparan sulfate 3-O-sulfotransferase 5 without encoding N-terminal membrane domain" denotes the protein comprising (preferably consisting of) the amino acid sequence as depicted in the sequence depicted in SEQ ID NO: 21; the term "heparan sulfate 6-O-sulfotransferase 1 without encoding N-terminal membrane domain" denotes the protein comprising (preferably consisting of) the amino acid sequence as depicted in the sequence depicted in SEQ ID NO: 23; the term "phosphoglucomutase 1" denotes the protein comprising (preferably consisting of) the amino acid sequence as depicted in the sequence depicted in SEQ ID NO: 25; the term "phosphoglucomutase 2" denotes the protein comprising (preferably consisting of) the amino acid sequence as depicted in the sequence depicted in SEQ ID NO: 27; the term "UDP-glucose pyrophosphorylase 1" denotes the protein comprising (preferably consisting of) the amino acid sequence as depicted in the sequence depicted in SEQ ID NO: 29; the term "glutamine-fructose-6-phosphate amidotransferase 1" denotes the protein comprising (preferably consisting of) the amino acid sequence as depicted in the sequence depicted in SEQ ID NO: 31; the term "phosphoacetylglucosamine mutase 1" denotes the protein comprising (preferably consisting of) the amino acid sequence as depicted in the sequence depicted in SEQ ID NO: 33; the term " methionine requiring protein 3" denotes the protein comprising (preferably consisting of) the amino acid sequence as depicted in the sequence depicted in SEQ ID NO: 35; the term "methionine requiring protein 14" denotes the protein comprising (preferably consisting of) the amino acid sequence as depicted in the sequence depicted in SEQ ID NO: 37; the term "methionine requiring protein 22" denotes the protein comprising (preferably consisting of) the amino acid sequence as depicted in the sequence depicted in SEQ ID NO: 39; the term "chitin-synthase 2" denotes the protein comprising (preferably consisting of) the amino acid sequence as depicted in the sequence depicted in SEQ ID NO: 41; the term "chitin-synthase 3" denotes the protein comprising (preferably consisting of) the amino acid sequence as depicted in the sequence depicted in SEQ ID NO: 43; the term "3'-phosphoadenylyl-sulfate reductase " denotes the protein comprising (preferably consisting of) the amino acid sequence as depicted in the sequence depicted in SEQ ID NO: 4.

The nucleic acid sequence comprises regulatory regions (herein also denoted as regulatory polynucleotide sequences or regulatory sequences), transcribed regions (such as regions that code for proteins, but also regions that are transcribed but do not code for proteins, such as introns) and/or other functional sequence regions. Examples of regulatory polynucleotide sequences are promoters, enhancers, terminators, silencers, IRES-sequences, ribosome-binding sites, and sequences stabilizing or destabilizing the mRNA by secondary structures. Within the meaning of the present invention, the term "promoter" demotes a DNA sequence that directs the transcription of a (structural) gene. Typically, a promoter is located in the 5'-region of a gene, proximal to the transcriptional start site of a (structural) gene. Promoter sequences may be constitutive, inducible or repressible. Within the meaning of the present invention, the term "native promoter" denotes a single endogenous sequence of DNA downstream of an endogenous gene that directs transcription of said gene. In a particular embodiment of the present invention, the native promoter is native CHS3 promoter (pCH3) which is encoded by a nucleotide (nt) sequence comprising, preferably consisting of, nucleotide (nt) sequence SEQ ID NO: 47, directing the transcription of chitin synthase 2/3. In another particular embodiment of the present invention, the native promoter is native MET16 promoter (pMET16) which is encoded by a nucleotide (nt) sequence comprising, preferably consisting of, nucleotide (nt) sequence SEQ ID NO: 48, directing the transcription of 3'-phosphoadenylyl-sulfate reductase.

Within the meaning of the present invention, the expression "strong promoter" denotes a gene-regulating promoter that results in comparably high levels of transcription, and thereby results in comparably high levels of corresponding protein, when compared to a gene-regulating promoter that results in lower levels of transcription and thus lower levels of corresponding protein. The latter promoter is then referred to as a "weak" promoter. In a preferred embodiment, the strong promoter is the promoter of TDH3 (pTDH3) which is encoded by a nucleotide (nt) sequence comprising, preferably consisting of, nucleotide (nt) sequence SEQ ID NO:50, the promoter of TEF1 (pTEF1) which is encoded by a nucleotide (nt) sequence comprising, preferably consisting of, nucleotide (nt) sequence SEQ ID NO: 51, or the promoter of PGK1 (pPGK1) which is encoded by a nucleotide (nt) sequence comprising, preferably consisting of, nucleotide (nt) sequence SEQ ID NO: 52. In a preferred embodiment, the weaker promoter is KEX2 promoter (pKEX2) which is encoded by a nucleotide (nt) sequence comprising, preferably consisting of, nucleotide (nt) sequence: SEQ ID NO: 49.

Within the meaning of the present invention, the term "recombinant (microorganism/yeast/strain)" denotes a cell/strain containing nucleic acids which is the result of a different genetic combination and ultimately results in the formation of unique gametes with chromosomes that are different from those in the parents. In particular a recombinant cell or a recombinant strain may comprise nucleic acid not present in a corresponding wildtype cell or wildtype strain, which nucleic acid has been introduced into that strain (cell) using recombinant DNA techniques. Recombinant DNA techniques are described elsewhere herein. In particular a recombinant strain maybe capable of production of targeting substances of which the wildtype stain is not capable. For instance, the recombinant S. cerevisiae comprises a heterologous AtUGD1 gene enable the expression of UDP-glucose dehydrogenase; the recombinant S. cerevisiae comprises a heterologous PmHS1 and/or PmHS2 gene enable the expression of heparosan synthase 1 and/or heparosan synthase 2; the recombinant S. cerevisiae comprises a heterologous PmHS1_NES and/or PmHS2_NES gene enable the expression of heparosan synthase 1 and/or heparosan synthase 2 specifically in cytosol, rather than localizing in the yeast nucleus; the recombinant S. cerevisiae capable of heparason production comprises a heterologous genes(s) encoding NDST1-ΔMD , and/or C5-epi-ΔMD, and/or HS2ST1-ΔMD, and/or HS3ST1-ΔSD, and/or HS3ST3 A1-ΔMD, and/or HS3ST5-ΔMD, and/or HS6ST1-ΔMD and/or an respective like-form thereof enable the expression of N-deacetylase 1 and/or N-sulfotransferase 1, and/or C5-epimerase, and/or heparan sulfate 2-O-sulfotransferase 1, and/or heparan sulfate 3-O-sulfotransferase 1, and/or heparan sulfate 3-O-sulfotransferase 3 A1, and/or heparan sulfate 3-O-sulfotransferase 5, and/or heparan sulfate 6-O-sulfotransferase 1 respectfully and specifically in cytosol rather than localizing in the endoplasmic reticulum or Golgi apparatus.

Within the meaning of the present invention, the term "wildtype" denotes that a certain matter is present in its natural occurring state, and thus exerts its natural function and/or activity. As used herein, a "certain matter" can for instance be an organism such as a microorganism, a cell, or, preferably, a certain yeast strain such as S. cerevisiae. The wildtype state, or wildtype form, respectively, of a certain matter is distinguishable from the recombinant forms of said matter.

Within the meaning of the present invention, the expression "increased amount" (e.g. of UDP-GlcA (UDP-D-glucuronic acid), UDP-GlcNAc (UDP-N-acetyl-D-glucosamine), or PAPS (3'-phospho-adenylyl-sulfate)) denotes that the amount of the respective matter (e.g. precursor) is at least 103% per weight of the dry substance of the matter, preferably at least 105% per weight of the dry substance of the matter, more preferably at least 110% per weight of the dry substance of the matter, even ore preferably at least 120% per weight of the dry substance of the matter, even more preferably at least 130% per weight of the dry substance of the matter, and most preferably at least 140% per weight of the dry substance of the matter, compared to the amount produced by the corresponding wild type cell/cell strain, with the amount produced by the wildtype cell/cell strain being 100%.

Within the meaning of the present invention, the term "heterologous", e.g. heterologous proteins or genes, denote proteins or genes derived from a different organism. In particular, the proteins encoded by heterologous genes denote that said proteins was initially cloned from or derived from a different cell type or a different species from the recipient, preferably different from the wildtype strains. For instance,

AtUGD gene, PmHS1 gene, PmHS2 gene, PmHS1_NES gene, PmHS2_NES gene, NDST gene, C5-epi gene, HS2ST gene, HS3ST1 gene, HS3ST3 gene, HS3ST5 gene, and HS6ST gene as well as the respective proteins encoded therefrom are heterologous genes and proteins for the wildtype S. cerevisiae. Within the meaning of the present invention, the term "homologous" denotes "of the same kind" or alike, having corresponding parts, similar structures, or the same anatomical positions, and that may indicate a common origin. For instance, chitin synthases CHS 2 and CHS3 are homologous enzymes; phosphoglucomutase PGM 1 and PGM 2 are homologous enzymes; heparosan synthases PmHS1 and PmHS2 are homologous enzymes; heparan sulfate 3-O-sulfotransferase 1, heparan sulfate 3-O-sulfotransferase 3 A1, and heparan sulfate 3-O-sulfotransferase 5 are homologous enzymes.

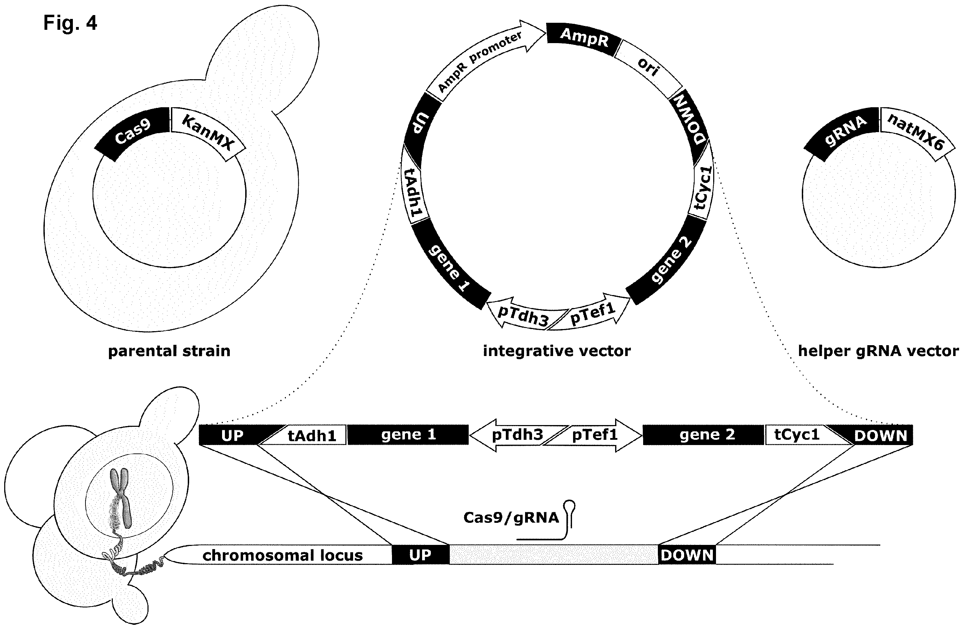

The introduction (or integration) of heterologous genes into a genome can be carried out according to any suitable method by applying recombinant DNA techniques that is known to a person skilled in the art, for example by applying the technique of marker-free CRISPR-Cas9 aided gene integration (Fig. 4).

Within the meaning of the present invention the term "stable integration" / "stable gene integration" denotes the integration of a fragment of DNA into the host genome (i.e. the integration into one of the host chromosomes), thereby ensuring the maintenance of the integrated DNA fragment through many generations.

Within the meaning of the present invention, the term "marker-free", e.g. marker free gene integration refers to the absence of a selection marker (such as kanamycin). Marker-free gene integration is advantageous for industrial strains.

Within the meaning of the present invention, the term "endogenous", e.g. endogenous proteins or genes denote proteins or genes originate from within a system such as an organism, tissue, or cell. In particular, the proteins encoded by endogenous genes denote that said proteins was initially cloned from or derived from within the wildtype strains. For instance, HXT1/2/4/5/7 gene, GLK1 gene, PGM1/2 gene, UGP1 gene, PGI1 gene, GFA1 gene, GNA1 gene, PCM1 gene, QRI1 gene, CHS2/3 gene, Sul1/2 gene, MET3 gene, MET14 gene, MET16 gene, and MET22 gene as well as the respevtive proteins encoded therefrom are endogenous genes and proteins for the wildtype S. cerevisiae.

Within the meaning of the present invention, recombinant DNA techniques known to the person skilled in the art are methods of molecular cloning (e.g. preparation of plasmid vectors, digestion of plasmid vectors, preparation of DNA by polymerase chain reaction, assembly of recombinant DNA through Gibson assembly with T5 exonuclease, Phusion polymerase, Taq ligase), methods through which recombinant DNA is introduced into host organism (e.g. transformation), methods through which recombinant DNA is integrated into host genome (e.g. CRISPR/Cas9 mediated homology-directed repair) and methods through which organisms are screened for the possession of correct recombinant DNA (e.g. selection using antibiotic resistance, colony PCR, Sanger sequencing).

Within the meaning of the present invention, the term "recombinant DNA" denotes molecules of DNA formed by recombinant DNA techniques to combine DNA from different sources.

Within the meaning of the present invention, the term "chassis" denotes a platform that acts as framework and supports for biological parts and components, preferably a recombinant yeast, preferably S. cerevisiae, that acts as framework and supports for production and/or modification of targeting substances. In a preferred embodiment of the present invention, the recombinant yeast chassis is a recombinant S. cerevisiae strain that is capable of providing high cytosolic concentrations of precursors UDP-GlcA and UDP-GlcNAc for heparosan synthesis, and optionally high cytosolic concentration of precursor PAPS for herapan sulfates synthesis. Within the meaning of the present invention, the term "precursor" denotes a substance, cell, or cellular component from which another substance, cell, or cellular component is formed.

Within the meaning of the present invention the term "increased", e.g. "increased" transcription denotes that the total transcription of genes encoding e.g. GFA1, PCM1, PMG1/2, UGP1, MET3, MET14, or MET22 protein in the recombinant strains is at least 3%, preferably at least 5%, preferably at least 10%, more preferably at least 15%, even more preferably at least 20% more than the total transcription of genes encoding e.g. GFA1, PCM1, PMG1/2, UGP1, MET3, MET14, MET22 protein in the wildtype form. The "increased" (cytosolic) concentration or "increased" amount denotes that the total (cytosolic) concentration or amount of a matter (e.g. D-Glucosamine-6-P, N-acetyl-Dglucosamine-1-P, D-Glucose-1-P, UDP-D-glucose, UDP-GlcA, UDP-GlcNAc, APS, PAPS, AMP, heparosan, heparan sulphates, or heparin) is at least 5%, preferably at least 10%, more preferably at least 15%, even more preferably at least 20% more than the total concentration or amount of the wildtype form of this matter (e.g. D-Glucosamine-6-P, N-acetyl-Dglucosamine-1-P, D-Glucose-1-P, UDP-D-glucose, UDP-GlcA,UDP-GlcNAc, APS, PAPS, AMP, Haparosan, heparin sulphates, or heparin).

Within the meaning of the present invention the term "decreased", e.g. "decreased" transcription denotes that the total transcription of genes encoding e.g. CHS2/3, or MET16 protein in the recombinant strains is at least 5%, preferably at least 10%, more preferably at least 15%, even more preferably at least 20% less than the total transcription of genes encoding e.g. CHS2/3, or MET16 in the wildtype form. The "decreased" (cytosolic) concentration or "decreased" amount denotes that the total (cytosolic) concentration or amount of a matter (e.g. chitin, or PAP) is at least 5%, preferably at least 10%, more preferably at least 15%, even more preferably at least 20% less than the total concentration or amount of the wildtype form of this matter (e.g. chitin, or PAP).

Within the meaning of the present invention, the term "transcription", e.g. "transcription of a gene" denotes the process by which the information in a strand of DNA is copied into a new molecule of messenger RNA (mRNA) which carries the genetic information needed to make proteins in a cell.

Within the meaning of the present invention, the term "expression", e.g. "expression of a gene" denotes a process that involves making an RNA copy of the DNA code (transcription) and translating information carried by nucleic acids to give the sequences of amino acids that make up said protein.

Within the meaning of the present invention, the term "overexpression" denotes an (e.g., artificially) excessive expression of a gene resulting in abnormally large amounts of a protein or other substance, compared to the expression of said gene in a corresponding wildtype cell.

Within the meaning of the present invention, the term "downregulation" denotes a process which can be natural by which a cell decreases the quantity of a cellular component, such as RNA, protein, or metabolite, in response to an external stimulus, compared to the respective quantity of said cellular component in a corresponding wildtype cell.

An "overexpression" or "downregulation" can be arrived at by any suitable means that is known to a person skilled in the art. For example, it can be arrived at by applying amendments to a nucleic acid encoding a specific (desired) gene product. Such amendments can for example be the introduction of a point mutation, e.g. substitution, insertion, or deletion of a single or more nucleotides; by partial or complete deletion; and/or by replacement by a different, e.g., heterologous, nucleotide sequence; or, with regard to "overexpression", by introducing one or more additional gene copies of the gene to be overexpressed; or, with regard to "downregulation", by reducing the number of copies of the gene to be "downregulated".

It is also possible that the "overexpression" or "downregulation" is arrived at by introduction of a point mutation, e.g., substitution, insertion, or deletion of a single or more nucleotides in regulatory polynucleotide sequence(s) of the affected gene(s); by partial or complete deletion or replacement of regulatory polynucleotide sequences of the affected gene(s); and/or by replacement of the regulatory polynucleotide sequence(s) of the affected gene(s), by different, e.g. heterologous, nucleotide sequences.

Regulatory sequences are sequences that control the expression of certain genes, for example promoters, enhancers, terminators, silencers, IRES-sequences, ribosome-binding sites, and sequences stabilizing or destabilizing the mRNA by secondary structures.

In a preferred embodiment, with regard to "overexpression", endogenous transaminase GFA1 and P-acetyl-glucosamine mutase PCM1 are overexpressed by chromosomal integration of an additional gene copy with a strong promoter upstream of said gene. In a further preferred embodiment, sulfate adenylyltransferase (ATP-sulfurylase) MET3, adenylyl-sulfate kinase MET14, and adenosine 3',5'-bisphosphate nucleotidase MET22 are overexpressed by chromosomal integration of an additional gene copy with a strong promoter upstream of said gene. Strong promoters are e.g. the promoter of TDH3 (pTDH3), the promoter of TEF1 (pTEF1) or the promoter of PGK1 (pPGK1). Overexpression can also be achieved by dead Cas9 (dCas9) mediated transcriptional programming. Here, dCas9 can be modified to act as transcription factor for specific genes, thereby increasing expression of these genes.

In a preferred embodiment, with regard to "downregulation", a gene encoding the enzyme chitin-synthase, preferably CHS2/3, is downregulated by a comparably weaker promoter, preferably by replacing the native promoter of CHS2/3 by KEX2 promoter, to the effect that transcription of CHS2/3 gene is decreased therefore the amount of the chitin-synthase is decreased; In another preferred embodiment, a gene encoding the enzyme 3'-phosphoadenylyl-sulfate reductase, preferably MET16 is downregulated by a comparably weaker promoter, preferably by replacing the native promoter of CHS2/3 with the comparably weaker KEX2 promoter (pKEX2), to the effect that the transcription of MET16 gene is reduced therefore the amount of the 3'-phosphoadenylyl-sulfate reductase is decreased. Downregulation can also be achieved by dead dCas9 mediated transcriptional programming. Here, dCas9 can be modified to act as transcription factor for specific genes, thereby decreasing expression of these genes. Alternatively, genes such as CHS2/3 can be knocked-out/deleted from the yeast genome to achieve down regulation, as long as the deletion is not lethal for the yeast.

Within the meaning of the present invention, the term "upregulation" denotes a process which can be natural by which a cell increases the quantity of a cellular component, such as RNA, protein or metabolite, in response to an external stimulus. For instance, the amount of UDP-GlcNAc produced in S. cerevisiae is upregulated by treating the culture of S. cerevisiae with mating pheromones.

Within the meaning of the present invention, the term "modification" denotes a chemical or structural alteration in the structure of a molecule by enzymatic means, resulting in a change in the properties of that molecule.

Within the meaning of the present invention, the term "polymerisation" denotes the act or process wherein monomeric molecules join together to form a polymer or a 3D network. As used herein, polymerisation occurs through a chemical reaction that ends up in creating a polymer as monomers link through chemical bonds. Polymers produced by a living organism are referred to as biopolymers. They include proteins and polypeptides, polynucleotides, and polysaccharides. Polysaccharides are biopolymers that are linked by the coming together of monomeric sugar units joined together by glycosidic bonds.

Within the meaning of the present invention, the term "titer" refers to a produced concentration of a compound.

Within the meaning of the present invention, the term "-like" or "like-form", e.g. "a protein/gene-like", denotes a protein/gene exhibits essentially the same function and activity as the protein/gene as itself. For instance, the HS3TS1-like protein/gene exhibits essentially the same function and activity as the HS3TS1 protein/gene. As used herein, "essentially the same" defines a deviation of up to 20%, preferably of up to 10%, more preferably of up to 7% and even more preferably of up to 3% of a given value.

Within the meaning of the present invention, the term "isoform", e.g. "isoform of a protein" denotes a member of a set of highly similar proteins that originate from a single gene or gene family and are the result of genetic differences. Many isoforms perform the same or similar biological roles, some however have unique functions. For instance, heparosan synthases isoform 2 (PmHS2) exhibits less dependency on polysaccharide primers while heparosan synthases isoform 1 (PmHS1) possesses a 3-fold faster heparosan elongation rate leading to larger molecular-weight polymers [3].

For instance, HS3ST isoform 1 (HS3ST1) catalyses the sulfate transfer exclusively to the 3-OH of glucosamines that are linked to either GlcA or IdoA. In contrast to HS3ST1, HS3ST3 requires an adjacent 2-O-sulfated IdoA (IdoA2S) residue for the sulfate transfer. HS3ST5's activity is independent of the linked uronic acid residues [4].

Within the meaning of the present invention, the term "microorganism" refers to an organisms that can only be seen under a microscope. Microorganisms include bacteria, protozoa, algae and fungi.

Within the meaning of the present invention, the term "yeast" or "yeast cell" refers to a phylogenetically diverse group of single-celled fungi, most of which are in the division of Ascomycota and Basidiomycota. The budding yeasts ("true yeasts") are classified in the order Saccharomycetales, with Saccharomyces cerevisiae as the most well-known species. Within the meaning of the present invention, the yeast or yeast cell can be selected from the group of yeasts with the genus Saccharomyces, Pichia, Yarrowia, Kluyveromyces, Candida, Rhodotorula, Rhodosporidium, Cryptococcus, Trichosporon and Lipomyces, such as Saccharomyces cerevisiae, Saccharomyces boulardi, Pichia pastoris, Kluyveromyces marxianus, Cryptococcus albidus, Lipomyces lipofera, Lipomyces starkeyi, Rhodosporidium toruloides, Rhodotorula glutinis, Trichosporon pullulan and Yarrowia lipolytica.

Within the meaning of the present invention, the term "glycosaminoglycan" denotes a linear polysaccharide comprised of repeating disaccharide (i.e. two-sugar) units of an uronic sugar and an amino sugar or an uronic sugar and galactose. Heparosan, keratan, chondroitin, dermatan, hyaluronic acid, and their sulfated derivatives are examples of glycosaminoglycans. Within the meaning of the present invention, the term "heparosan" denotes a glycosaminoglycan with the chemical composition: [→4) β-D-glucuronic acid (GlcA) (1→4) *N*-acetyl-α-D-glucosamine (GlcNAc) (1→)] . n

Within the meaning of the present invention, the term "heparosan synthase" refers to an enzyme with UDP-GlcA glucosyltransferase and/or UDP-GlcNAc glucosyltransferase activity that by itself or together with other enzymes is capable of heparosan synthesis. Within the meaning of the present invention, the term "hyaluronic acid synthase" refers to an enzyme with UDP-GlcA glucosyltransferase and/or UDP-GlcNAc glucosyltransferase activity that by itself or together with other enzymes is capable of hyaluronic acid synthesis.

Within the meaning of the present invention, the term "heparan sulfate" denotes a glycosaminoglycan like heparosan that is additionally sulfated at the 3-O and/or 6-O and/or nitrogen of the glucosamine residue and/or sulfated at the 2-O of the glucuronic acid residue. Further, the glucuronic acid residues might be partially epimerised to iduronic acid residues. Within the meaning of the present invention, the term "heparin" denotes a heparan sulfate that inhibits blood coagulation. Within the meaning of the present invention, the term "sulfation pattern" denotes a specific sequence of modifications (e.g. deacetylation, N-sulfation, O-sulfation) of the uronic sugar and amino sugar residues of the glycosaminoglycan.

### Detailed description of the invention

The present invention is now described in more details by preferred embodiments and examples which are however presented for illustrative propose only and shall not be understood as limiting the scope of the present invention in any way.

The conventional production of heparin and its analogs relies on extraction from animal tissue, in particular porcine intestines, which may lead to contamination of the drug and shortages during animal disease outbreaks. While bacterial enzymes can be employed for synthesizing heparosan (precursor of heparin sulfates and heparin), all enzymes required for modifying heparosan into heparan sulfates and heparin biosynthesis are exclusively of vertebrate origin. Since enzymes of eukaryotic origin are generally more stable and functional when expressed in eukaryotic hosts, yeast cells are potent cell factory candidates which are better suited for biosynthesis of heparin sulfates and heparin than bacterial system. Furthermore, yeast cell factories allow for precise control over the kind and extent of enzymatic modifications of heparosan through a tightly controlled enzyme expression process, thereby providing a solution for industrial scale production of homogeneous, structurally well-defined heparan sulfates.

The present invention provides a superior production platform for glycosaminoglycans (GAG), in particular to the production of heparosan, its sulfated derivatives such as heparan sulfates and heparin, as well as an industrial scale production of heparan sulfate with defined sulfation patterns which can be used for a wide range of medical and/or research applications, using eukaryotic cell factories, specifically yeast cell factories. In a preferred embodiment, the yeast cells are S. cerevisiae cells.

The present invention is based on three units including Unit (1), referring to engineering optimized yeast chassis with efficient substrate utilization and capable of producing high precursor concentrations for biosynthesis of heparosan, Unit (2), referring to engineering recombinant yeast strains capable of producing high titers of heparosan, and Unit (3), referring to engineering recombinant yeast strains capable of producing high titers of heparan sulfates, heparin, and its analogs with defined sulfation patterns. In the following, these three units will be described in detail.

The invention is exemplified with S. cerevisiae; however, it is possible to use any type of yeast cell, as disclosed elsewhere herein.

### Unit (1) Optimised Chassis Engineering

To establish yeast cell factories as economically viable production platforms for heparosan and heparan sulfates, the present invention provides a recombinant yeast chassis that can efficiently utilize comparably cheap substrates (such as D-glucose, (NH4)SO4) and achieves high/increased cytosolic precursor concentrations of UDP-D-glucuronic acid (UDP-GlcA) and UDP-acetyl-D-glucosamine (UDP-GlcNAc) for heparosan synthesis, as well as high/increased cytosolic precursor concentration of 3'-phosphoadenylyl-sulfate (PAPS) for modification of heparosan into heparan sulfates, such as heparin. The disjointed biosynthesis pathways for the UDP-sugars enabled separate optimisations in a modular pathway engineering approach (cf. Fig. 1).

In detail, it is the scope of the first Unit (1) to engineer the modular pathway in order to attain high/increased cytosolic concentrations of UDP-GlcA and/or UDP-GlcNAc, preferably of both, in the yeast chassis. As it is known that UDP-GlcA is not endogenously produced in yeast, a heterologous gene encoding UDP-glucose dehydrogenase which directly leads to UDP-GlcA from UDP-glucose (the direct precursor of UDP-GlcA) is designed to integrate in the parent yeast. Therefore, in a preferred embodiment of the present invention, a heterologous gene encoding UDP-glucose dehydrogenase is integrated in the parent yeast, preferably S. cerevisiae S288C, wherein the heterologous gene encoding UDP-glucose dehydrogenase is AtUGD1 [SEQ ID NO: 2] derived from Arabidopsis thaliana, yielding a subsequent recombinant strain ST_GNS-005 capable of UDP-GlcA production. Strain ST_GNS-005 can further be engineered to enable production of heparosan and/or heparan sulfates.

Even though the cytosolic concentration of UDP-glucose is innately large in S. cerevisiae, in order to further increase the cytosolic concentration of UDP-GlcA, the endogenous phosphoglucomutase and UTP-glucose-1-phosphat-uridylyltransferase (UDP-glucose pyrophosphorylase) of the yeast cell, which directly lead to UDP-glucose from D-glucose-6-P, are additionally designed to overexpress in the subsequent strain which is capable of UDP-GlcA production. Overexpression can be achieved by any suitable means that is known to a person skilled in the art. In one embodiment of the present invention, the overexpression of phosphoglucomutase in yeast cells can be achieved by chromosomal integration of an additional gene copy encoding the phosphoglucomutase together with a strong promoter, e.g. pTEF1 upstream of said gene.

In another embodiment of the present invention, the overexpression of UTP-glucose-1-phosphat-uridylyltransferase (UDP-glucose pyrophosphorylase) in yeast cells can be achieved by chromosomal integration of an additional gene copy encoding the UTP-glucose-1-phosphate-uridylyltransferase together with a strong promoter, e.g. pTDH3 upstream of said gene.

As disclosed herein, in a preferred embodiment, PGM1/2 and UGP1 are overexpressed in a subsequent recombinant strain which is capable of producing UDP-GlcA by simultaneous chromosomal integration of an additional gene copy of PGM2 together with a strong promoter pTEF1 as well as chromosomal integration of an additional gene copy of UGP1 together with a strong promoter pTDH3 into the parent strain expressing AtUGD1, preferably strain ST_GNS-005, thereby yielding a subsequent recombinant yeast strain ST_GNS-009 capable of producing an increased cytosolic concentration of UDP-GlcA. Strain ST_GNS-009 can further be engineered to enable production of heparosan and/or heparan sulfates.

Mainly as a precursor of chitin, UDP-GlcNAc is synthesised in S. cerevisiae from fructose-6-P in four steps: fructose-6-P is first transaminated by transaminase encoded by GFA1 gene to glucosamine-6-P utilising L-glutamine as nitrogen donor. Glucosamine-6-P is subsequently acetylated by acetylase encoded by GNA1 gene to N-acetyl-glucosamine-6-P and then converted to N-acetyl-glucosamine-1-P by a P-acetyl-glucosamine mutase encoded by PCM1 gene. In the last synthesis step, N-acetyl-glucosamine-1-P is converted into UDP-GlcNAc by a UDP-GlcNAc pyrophosphorylase encoded by QRI1 gene. It has been shown that S. cerevisiae cultures treated with mating pheromones, which upregulate UDP-GlcNAc and consequently chitin production, lead to increased transcription of GFA1 and PCM1 genes. This dynamic suggests that glutamine--fructose-6-phosphate aminotransferase 1 (GFA1) and glucosamine-phosphate mutase 1 (PCM1) are bottlenecks in the UDP-GlcNAc pathway. Therefore, the endogenous GFA1 and PCM1 genes are engineered to be overexpressed in order to yield an increased cytosolic concentration of UDP-GlcNAc. In an embodiment of the present invention, the overexpression of GFA1 in the yeast cells can be achieved by chromosomal integration of an additional gene copy of GFA1 together with a strong promoter pTDH3. In one embodiment of the present invention, the overexpression of PCM1 in the yeast cells can be achieved by chromosomal integration of an additional gene copy of PCM1 together with a strong promoter pTEF1.

As disclosed herein, in some embodiments of the present invention, GFA1 and PCM1 are overexpressed by simultaneous chromosomal integration of an additional gene copy of GFA1 together with a strong promoter pTDH3 as well as chromosomal integration of an additional gene copy of PCM1 together with a strong promoter pTEF1 in any one of the previously engineered subsequent strains which are capable of producing UDP-GlcA, thereby yielding a recombinant yeast strain capable of producing both UDP-GlcA and UDP-GlcNAc. In a preferred embodiment, GFA1 and PCM1 are overexpressed in the recombinant strain ST_GNS-005 by simultaneous chromosomal integration of an additional gene copy of GFA1 together with a strong promoter pTDH3 as well as chromosomal integration of an additional gene copy of PCM1 together with a strong promoter pTEF1, thereby yielding a subsequent recombinant yeast strain ST_GNS-008 that is capable of producing UDP-GlcA and an increased cytosolic concentration of UDP-GlcNAc. Strain ST_GNS-008 can further be engineered in order to enable the production of heparosan and/or heparan sulfates. In a more preferred embodiment, GFA1 and PCM1 are overexpressed in the recombinant strain ST_GNS-009 simultaneous chromosomal integration of an additional gene copy of GFA1 together with a strong promoter pTDH3 as well as chromosomal integration of an additional gene copy of PCM1 together with a strong promoter pTEF1, thereby yielding a subsequent recombinant yeast strain ST_GNS-010 that is capable of producing an increased cytosolic concentration of both UDP-GlcA and UDP-GlcNAc. Strain ST_GNS-010 can further be engineered in order to enable the production of heparosan and/or heparan sulfates.

In addition, to prevent consumption of cytosolic UDP-GlcNAc, the transcription of CHS3 gene encoding chitin synthase III is also designed to be downregulated, since chitin synthase III synthesises ca. 90% of chitin in yeast cell, particular in S. cerevisiae. The downregulation of transcription of CHS3 gene can be achieved by replacing the native CHS3 promoter downstream of the CHS3 gene in S. cerevisiae with a weaker promoter. As disclosed herein, in some embodiments of the present invention, the native CHS3 promoter is replaced by a weaker promoter which can be selected from the group consisting of pKEX2 and pHXT1, preferably pKEX2, in any one of the previously engineered subsequent strains, yielding a group of subsequent recombinant strains capable of producing UDP-GlcNAc with an increased cytosolic concentration when compared to the wildtype strain.

In a preferred embodiment, the native CHS3 promoter gene is replaced by the weaker promoter KEX2 in the recombinant strain ST_GNS-010, thereby yielding a subsequent recombinant strain ST_GNS-011 that is capable of producing both UDP-GlcA and UDP-GlcNAc with a further increased cytosolic concentration. Strain ST_GNS-011 can further be engineered in order to enable production of heparosan and/or heparan sulfates.

The second scope of Unit (1) is to increase the cytosolic 3'-phosphoadenylyl-sulfate (PAPS) concentration through a modular pathway engineering approach in recombinant yeast strains in addition to the previously improved (increased) UDP-sugar concentrations. To achieve that, sulfate adenylyltransferase (ATP-sulfurylase) MET3, the adenylyl-sulfate kinase MET14, and the adenosine 3',5'-bisphosphate nucleotidase MET22 are designed to be overexpressed in any one of the preceding engineered recombinant yeast strains. The overexpression of MET3 gene can be achieved by chromosomal integration of an additional gene copy of MET3 together with a strong promoter, e,g., pTDH3. The overexpression of MET14 gene can be achieved by chromosomal integration of an additional gene copy of MET14 together with a strong promoter, e.g., pTEF1. The overexpression of MET22 gene can be achieved by chromosomal integration of an additional gene copy of MET22 together with a strong promoter, e.g., pPGK1.

As disclosed herein, in some embodiments of the present invention, MET3, MET14, and/or MET22 gene(s) is/are overexpressed by in any one of the previously engineered subsequent strains, thereby yielding a group of subsequent recombinant yeast strains that capable of producing UDP-GlcA, UDP-GlcNAc, and PAPS. In a preferred embodiment, MET3 and MET14 genes are overexpressed in the recombinant strain ST_GNS-011 by simultaneous chromosomal integration of an additional gene copy of MET3 together with a strong promoter pTDH3 and MET14 together with a strong promoter pTEF1, thereby yielding a subsequent recombinant yeast strain ST_GNS-012 that is capable of producing an increased cytosolic concentration of UDP-GlcA, UDP-GlcNAc, and PAPS. Strain ST_GNS-012 can further be engineered in order to enable production of heparosan and/or heparan sulfates. In a more preferred embodiment, MET3, MET14, and MET22 genes are overexpressed in the recombinant strain ST_GNS-0011 by simultaneous chromosomal integration of an additional gene copy of MET3 together with a strong promoter pTDH3, MET14 together with a strong promoter pTEF1 and MET22 together with a strong promoter pPGK1, thereby yielding a subsequent recombinant yeast strain ST_GNS-013 that is capable of producing an optimised (increased) cytosolic concentration of UDP-GlcA, UDP-GlcNAc and PAPS. Strain ST_GNS-013 can further be engineered in order to enable production of heparosan and/or heparan sulfates.

Furthermore, in order to prevent consumption of cytosolic PAPS, it is also designed to downregulate the transcription of MET16 gene encoding endogenous 3'-phosphoadenylyl-sulfate reductase. The downregulation of transcription of MET16 gene can be achieved by replacing the native promoter of MET16 in the genome of the yeast with the comparably weaker promoter or by dead dCas9 mediated transcriptional programming or by knockout of the gene entirely.

As disclosed herein, in some embodiments of the present invention, MET16 gene is downregulated by replacing the native promoter of MET16 (pMET16) in the genome of the yeast with the comparably weaker KEX2 promoter (pKEX2) in any one of the previously engineered subsequent strains, thereby yielding a group of subsequent recombinant yeast strains capable of producing UDP-GlcA, UDP-GlcNAc, and PAPS. In a more preferred embodiment, MET16 gene is downregulated in the recombinant strain ST_GNS-013 by replacing the native promoter of MET16 (pMET16) in the genome of the yeast with the comparably weaker KEX2 promoter (pKEX2), thereby yielding a subsequent recombinant yeast strain ST_GNS-014 that is capable of producing an optimised cytosolic concentration of UDP-GlcA, UDP-GlcNAc, and PAPS. Strain ST_GNS-014 can be used as an optimised yeast chassis for further engineering to enable production of heparason and/or heparan sulfates.

### Unit (2) Engineering of Heparosan Producing Yeast Strains

Heparosan, a glycosaminoglycan consisting of repeating disaccharide units (-GlcA-β1,4-GlcNAc-al,4-), is the unmodified GAG precursor of heparin and other heparan sulfates. In mast cells, the biosynthesis of heparosan is initiated through the formation of a tetrasaccharide linkage region (-GlcA-β1,3-Gal-β1,3-Gal-β1,4-Xyl-O-Ser) on a specific Ser residue of a serglycin core protein. The formation of this linker is catalysed by four enzymes, each elongating the linker by one residue from the respective UDP-sugar. The tetrasaccharide linkage region serves as primer for the subsequent heparosan ([-GlcA-β1,4-GlcNAc-a1,4-]ₙ) synthesis by alternating addition of glucuronate and N-acetyl-glucosamine residues from their UDP-sugars. This polymerisation is executed by a hetero-oligomeric complex of the GlcA/GlcNAc transferases II EXT1 and EXT2 from the exotosin family. This complex eukaryotic pathway for heparosan biosynthesis requiring a number of different enzymes and UDP-sugars can be replaced with bacterial heparosan synthases requiring only UDP-GlcA and UDP-GlcNAc. While E. coli K5 expresses two genes, KfiC and KfiA, encoding for a UDP-GlcA glucosyltransferase and a UDP-GlcNAc glucosyltransferase respectively, Pasteurella multocida (P. multocida) expresses two highly homologous dual action heparosan synthases, PmHS1 and PmHS2. Even though both P. multocida enzymes display glucuronic acid and hexosamine transfer activity (dual-action), their different catalytic preferences suggest distinct roles for heparosan synthesis: PmHS2 exhibits less dependency on polysaccharide primers while PmHS1 possesses a 3-fold faster heparosan elongation rate leading to larger molecular-weight polymers. When PmHS2 was heterologously expressed in Bacillus megaterium, heparosan titers of about 250 mg/L in shake flasks and 2.74 g/L in bioreactors were achieved.

Therefore, genes encoding heparosan synthesas PmHS1 and/or PmHS2 is/are designed to integrate into the recombinant yeast strain which is capable of producing UDP-GlcA, and UDP-GlcNAc, thereby achieving recombinant yeast strains capable of heparosan production. Thus, in some embodiments of the present invention, genes encoding heparosan synthesas PmHS1 and/or PmHS2, preferably PmHS1 and PmHS2 is/are integrated into the genome of any one of the previously engineered yeast strains, yielding a group of subsequent recombinant yeast strains capable of producing heparosan. In a preferred embodiment, PmHS1 gene is integrated in the recombinant strain ST_GNS-005, thereby yielding a subsequent recombinant yeast strain ST_GNS-001 capable of heparosan production. Strain ST_GNS-001 can further be engineered in order to enable production of heparan sulfates. In another preferred embodiment, PmHS2 gene is integrated in the recombinant strain ST_GNS-005, thereby yielding a subsequent recombinant yeast strain ST_GNS-02 capable of heparosan production. Strain ST_GNS-002 can further be engineered in order to enable production of heparan sulfates.

However, it was observed that the bacterial enzymes PmHS1 and PmHS2 tend to localize in the yeast nucleus, thereby impeding efficient heparosan biosynthesis. To counteract the nuclear localisation, the nuclear export sequence (NES) of S. cerevisiae's Hsp70 was fused to the C-termini of the heparosan synthases PmHS1 and PmHS2 in order to achieve a high cytosolic concentration of heparosan synthases. When PmHS1_NES and PmHS2_NES were expressed in S. cerevisiae, improved heparosan production was observed, particularly for the PmHS1_NES expressing strain. Co-expression of PmHS1_NES and PmHS2_NES further improved heparosan titer, which is likely due to different characteristics of the isoforms: PmHS2 is better synthesises heparosan primers while PmHS1 is the superior heparosan synthase for elongation.

Therefore, in some embodiments of the present invention, PmHS1_NES and/or PmHS2_NES genes, preferably PmHS1_NES and PmHS2_NES genes are integrated into the genome of any one of the previously engineered yeast strains, thereby yielding a group of subsequent recombinant yeast strains that are capable of expressing heparosan synthases PmHS1 and/or PmHS2, preferably PmHS1 and PmHS2 specifically in cytosol, rather than localizing in the yeast nucleus, thereby increasing the production of heparosan. In a preferred embodiment, PmHS1_NES gene is integrated into the genome of the recombinant strain ST_GNS-005, yielding a subsequent recombinant yeast strain ST_GNS-003 that is capable of producing an increased cytosolic concentration of heparosan. Strain ST_GNS-003 can further engineered to enable production of heparan sulfates. In another preferred embodiment, PmHS2_NES gene is integrated into the genome of the recombinant strain (ST_GNS-005), thereby yielding a subsequent recombinant yeast strain ST _GNS-004 that is capable of producing an increased cytosolic concentration of heparosan. Strain ST_GNS-004 can further be engineered in order to enable production of heparan sulfates. In another preferred embodiment, PmHS1_NES gene and PmHS2_NES gene are integrated into the genome of the recombinant strain ST_GNS-005, thereby yielding a subsequent recombinant yeast strain ST_GNS-007 that is capable of producing a further increased cytosolic concentration of heparosan. Strain ST_GNS-007 can further be engineered in order to enable production of heparan sulfates. In a more preferred embodiment, PmHS2_NES gene is integrated into the genome of the recombinant strain ST_GNS-014, thereby yielding a subsequent recombinant yeast strain ST_GNS-015 that is capable of producing an even further increased cytosolic concentration of heparosan. Strain ST_GNS-015 can further be engineered in order to enable production of heparan sulfates. In an even more preferred embodiment, PmHS1_NES gene and PmHS2_NES gene are integrated into the genome of the recombinant strain (ST_ GNS-014), thereby yielding a subsequent recombinant yeast strain ST _GNS-016 that is capable of producing optimised (increased) cytosolic concentration of heparosan. Strain ST_GNS-016 can further be engineered in order to enable production of heparan sulfates.

### (3) Engineering Yeast Strains for the Production of Heparan Sulfates and Heparin

Following the biosynthesis of heparosan, modification of the polysaccharide by a number of enzymes yields many distinct biofunctional heparan sulfates (Fig. 2a). The two initial heparosan modification steps, N-deacetylation and PAPS-dependent N-sulfation of N-acetyl-glucosamine are carried out by the dual-action enzyme N-deacetylase-N-sulfotransferase (NDST). While four highly homologous vertebrate NDST isoforms are known, only NDST1 and NDST2 exhibit high N-deacetylation as well as N-sulfation activity. In contrast, isoform NDST3 shows high N-deacetylation but very low N-sulfation activity. The N-sulfated Glucosamine (GlcNS) is necessary for the subsequent C5-epimerization of the adjacent D-glucuronic acid (GlcA) residue to L-iduronic acid (IdoA) by C5-epimerase (C5-epi). Epimerization to L-iduronic acid increases the residue's affinity for heparan sulfate 2-O-sulfotransferase (HS2ST) 5-fold, promoting sulfate transfer from PAPS to the C2 of the L-idoronyl residue. In addition to heparosan modification by N-deacetylation, N-sulfation, C5-epimerization and 2-O-sulfation, three isoforms of heparan sulfate 6-O-sulfotransferases (HS6ST) have been reported to catalyse the sulfate transfer from PAPS to the C6 of glucosamine residues of heparosan. All three isoforms show a prominent preference for GlcNS/GlcNAc residues immediately downstream of IdoA residues. The final modification of the heparan sulfate, the sulfate transfer to the 3-OH of a glucosamine unit is rare in nature but crucial for many biological functions of the resulting GAG. The seven known heparan sulfate 3-O-sulfotransferase (HS3ST) isoforms can be classified by their ability to transfer a sulfate group to a glucosamine bound at its non-reducing end to either GlcA, IdoA, IdoA2S or a combination thereof (Fig. 2b). For instance, HS3ST isoform 1 (HS3ST1) catalyses the sulfate transfer exclusively to the 3-OH of glucosamines that are linked to either GlcA or IdoA. In contrast to HS3ST1, HS3ST3 requires an adjacent 2-O-sulfated IdoA (IdoA2S) residue for the sulfate transfer. HS3ST5's activity is independent of the linked uronic acid residues [4].

The large number of possible heparosan modifications, i.e. N-deacetylation, N-sulfation, C5-epimerization, 2-O-sulfation, 6-O-sulfation, 3-O-sulfation as well as the combination of the modifications, results in an abundance of different heparosan derivatives. The complexity of possible enzymatically catalysed modifications of heparosan and their combinations is further extended by the differing substrate preferences of enzyme isoforms. This is particularly true for HS3ST isoforms, whose dissimilar substrate preferences mediate various distinct sulfation patterns. This amount of structural information is translated into a plethora of biological functions, the scope of which has yet to be clarified.

Therefore, in some embodiments of the present invention, gene(s) encoding N-deacetylase, and/or N-deacetylase-N-sulfotransferase, and/or C5-epimerase, and/or 2-O-sulfotransferase, and/or 3-O-sulfotransferase, and/or 6-O-sulfotransferase is/are additionally integrated in the genome of any one of the previously engineered recombinant yeast strains which is capable of producing heparosan, thereby yielding a group of subsequent recombinant yeast strains that are capable of producing heparan sulfates with heterogeneously well-defined sulfation patterns. In a preferred embodiment, N-deacetylase is ND1 and/or ND1-like; N-deacetylase-N-sulfotransferase is NDST1 and/or NDST1-like; 3-O-sulfotransferase is HS3ST1, HS3ST3 A1, or HS3ST5, and/or a like-form of HS3ST1, HS3ST3 A1, or HS3ST5 respectively; 2-O-sulfotransferase is HS2ST1 and/or HS2ST1, and/or 6-O-sulfotransferase is HS6ST1 and/orHS6ST1-like.

Due to the usage of heparin as anticoagulant, the SP that causes antithrombine binding to the haparan sulfate (GlcNS6S-GlcA-GlcnS3S6s-IdoA2S-GlcNS6S) is well characterised. However, the biological functions of heparan sulfates with different SPs and their action mechanisms have been studied only recently. Initial research on the topic suggests that heparan sulfates with defined modifications and SPs mediate a broad range of biological functions, enabling their therapeutic applications as anti-cancer, anti-inflammatory, antimicrobial, anti-diabetic or anti-neurodegenerative drugs (Fig.3). The research on biological functions and therapeutic application of structurally well-defined heparan sulfates has been hampered by the absence of production capabilities.

Therefore, the present invention provides yeast cell factories that are capable of industrial-scaled production of heparan sulfates with distinct modifications and sulfation patterns. To achieve that, the required heterologous genes for the heparosan modifications are designed to integrate into the recombinant yeast strains which are capable of heparonsan production. Since many of the required enzymes are natively localized in the endoplasmic reticulum, the genes encoding the respective enzymes were truncated to exclude the N-terminal membrane domain (MD) or N-terminal signal peptide (SD). Thus, cytosolic localisation of the heterologous enzymes can be achieved.

Therefore, in some embodiments of the present invention, genes encoding N-deacetylase, and/or N-deacetylase-N-sulfotransferase, and/or C5-epimerase, and/or 2-O-sulfotransferase, and/or 3-O-sulfotransferase, and/or 6-O-sulfotransferase are truncated to exclude the N-terminal membrane domain (MD), wherein HS3ST1 is truncated to exclude the N-terminal signal domain/signal peptide (SD), is/are additionally integrated into the genome of any one of the preceding engineered yeast strains which is capable of producing heparosan, thereby yielding a group of subsequent recombinant yeast strains capable of expressing N-deacetylase-N-sulfotransferase, and/or C5-epimerase, and/or 2-O-sulfotransferase, and/or 3-O-sulfotransferase, and/or 6-O-sulfotransferase specifically in cytosol, rather than localizing in the yeast endoplasmic reticulum or Golgi apparatus, thereby increasing the production of heparan sulfates with heterogeneously well-defined sulfation patterns. In a preferred embodiment, N-deacetylase truncated to exclude the N-terminal membrane domain (MD) is ND1-ΔMD and/or a like-form of ND1--ΔMD; N-deacetylase-N-sulfotransferase truncated to exclude the N-terminal membrane domain (MD) is NDST1-ΔMD and/or a like-form of NDST1-ΔMD; 3-O-sulfotransferase truncated to exclude the N-terminal membrane domain (MD) is HS3ST3 A1-ΔMD, or HS3ST5-ΔMD, and/or a like-form of HS3ST3 A1-ΔMD, or HS3ST5-ΔMD; 3-O-sulfotransferase truncated to exclude the N-terminal signal domain/signal peptide (SD) is HS3ST1-ΔSD and/or a like-form of HS3ST1-ΔSD; 2-O-sulfotransferase truncated to exclude the N-terminal membrane domain (MD) is HS2ST1-ΔMD and/or a like-form of HS2ST1-ΔMD; 6-O-sulfotransferase truncated to exclude the N-terminal membrane domain (MD) is HS6ST1-ΔMD and/or a like-form of HS6ST1-ΔMD.

Furthermore, in a preferred embodiment, NDST1-ΔMD gene and C5-epi-ΔMD gene are additionally integrated into the genome of the recombinant strain ST_GNS-002, thereby yielding a subsequent recombinant yeast strain ST_GNS-006 that is capable of producing heparan-N-sulfate with iduronic acid residues. Strain ST_GNS-006 can further be engineered in order to enable the modification of heparan-N-sulfate with iduronic acid residues into heparan sulfates with heterogeneously well-defined sulfation patterns.

In a more preferred embodiment, NDST1-AMD gene and C5-epi-AMD gene are additionally integrated into the genome of the recombinant strain ST_GNS-007, thereby yielding a subsequent recombinant yeast strain ST_GNS-017 that is capable of producing heparan-N-sulfate with iduronic acid residues. Strain ST_GNS-017 can further be engineered in order to enable the modification of heparan-N-sulfate with iduronic acid residues into heparan sulfates with heterogeneously well-defined sulfation patterns.

In an even more preferred embodiment, NDST1-ΔMD gene and C5-epi-ΔMD gene are additionally integrated into the genome of the recombinant strain ST_GNS-016, thereby yielding a subsequent recombinant yeast strain ST _GNS-020 that is capable of producing optimised (increased) cytosolic concentration of heparan-N-sulfate with iduronic acid residues. Strain ST_GNS-020 can further be engineered in order to enable the modification of heparan-N-sulfate with iduronic acid residues into heparan sulfates with heterogeneously well-defined sulphation patterns.

In another even more preferred embodiment, NDST1-ΔMD gene is additionally integrated into the genome of the recombinant strain ST_GNS-016, thereby yielding a subsequent recombinant yeast strain ST_GNS-025 that is capable of producing optimised (increased) cytosolic concentration of heparan-N-sulfate. Strain ST_GNS-025 can further be engineered in order to enable the modification of heparan-N-sulfate into heparan sulfates with heterogeneously well-defined sulfation patterns.

To achieve the correct sulfation patterns specifically regarding 2-O-sulfation, 3-O-sulfation and 5-O-sulfation, the integration of genes encoding HS2ST, HS6ST, HS3ST1/HS3ST1-like, HS3ST3/HS3ST3-like, and HS3ST5/HS3ST5-like sulfate transferase is crucial.

In a preferred embodiment, HS2ST1-ΔMD gene and HS6ST1-ΔMD gene are additionally integrated into the genome of the recombinant strain ST_GNS-017, yielding a subsequent recombinant yeast strain ST_GNS-018 that is capable of producing N-, IdoA2-O, 6-O-sulfated heparan sulfate as depicted in formula (4) and/or heparan sulfate containing one or more sequences of residues as depicted in formula (4) (Fig. 3) which is capable of binding growth factor FGF-2 thereby inhibiting the binding of FGF-2 to endothelial cells.

In another preferred embodiment, HS3ST1-ΔSD gene is additionally integrated into the genome of the recombinant strain ST_GNS-018, thereby yielding a subsequent recombinant yeast strain ST_GNS-019 that is capable of producing N-, IdoA2-O, 3-O, 6-O-sulfated heparan sulfate as depicted in formula (1) (heparin) and/or formula (5) and/or heparan sulfate containing one or more sequences of residues as depicted in formula (1) (heparin) and/or formula (5) which is capable of binding antithrombine.

In another even more preferred embodiment, HS2ST1-ΔMD gene and HS6ST1-ΔMD gene are additionally integrated into the genome of the recombinant strain ST_GNS-020, thereby yielding a subsequent recombinant yeast strain ST_GNS-021 that is capable of producing optimised (increased) cytosolic concentration of N-, IdoA2-O, 6-O-sulfated heparan sulfate as depicted in formula (4) and/or heparan sulfate containing one or more sequences of residues as depicted in formula (4) (Fig. 3) which is capable of binding growth factor FGF-2 thereby inhibiting the binding of FGF-2 to endothelial cells.

In another preferred embodiment, HS3ST1-ΔSD gene is additionally integrated into the genome of the recombinant strain ST_GNS-021, thereby yielding a subsequent recombinant yeast strain ST_GNS-022 that is capable of producing optimised (increased) cytosolic concentration of N-, IdoA2-O, 3-O,6-O-sulfated heparan sulfate as depicted in formula (1) (heparin) and/or formula (5) and/or heparan sulfate containing one or more sequences of residues as depicted in formula (1) (heparin) and/or formula (5) which is capable of binding antithrombin.

In another preferred embodiment, HS3ST3 A1-ΔMD gene is additionally integrated into the genome of the recombinant strain ST_GNS-021, thereby yielding a subsequent recombinant yeast strain ST_GNS-023 that is capable of producing optimised (increased) cytosolic concentration of N-, IdoA2-O, 3-O,6-O-sulfated heparan sulfate as depicted in formula (9) and/or heparan sulfate containing one or more sequences of residues as depicted in formula (9)in Fig. 3.

In another preferred embodiment, HS3ST5-ΔMD gene is additionally integrated into the genome of the recombinant strain ST_GNS-021, thereby yielding a subsequent recombinant yeast strain ST_GNS-024 that is capable of producing optimised (increased) cytosolic concentration of N-, IdoA2-O, 3-O,6-O-sulfated heparan sulfate as depicted in formula (8) and/or heparan sulfate containing one or more sequences of residues as depicted in formula (8) in Fig. 3.

In another preferred embodiment, HS6ST1-ΔMD gene is additionally integrated into the genome of the recombinant strain ST_GNS-025, thereby yielding a subsequent recombinant yeast strain ST_GNS-026 that is capable of producing optimised (increased) cytosolic concentration of N-, 6-O-sulfated heparan sulfate as depicted in formula (3) and/or heparan sulfate containing one or more sequences of residues as depicted in formula (3) (Fig. 3) which is capable of optimally binding human cytomegalovirus.

In another preferred embodiment, HS3ST5-ΔMD gene is additionally integrated into the genome of the recombinant strain ST_GNS-026, thereby yielding a subsequent recombinant yeast strain ST_GNS-027 that is capable of producing optimised (increased) cytosolic concentration of N-, 3-O,6-O-sulfated heparan sulfate as depicted in formula (2) and/or heparan sulfate containing one or more sequences of residues as depicted in formula (2) (Fig. 3) which is capable of increasing Tau binding.

In another preferred embodiment, HS6ST1-ΔMD gene is additionally integrated into the genome of the recombinant strain ST_GNS-020, thereby yielding a subsequent recombinant yeast strain ST_GNS-028 that is capable of producing optimised (increased) cytosolic concentration of N-, 6-O-sulfated heparan sulfate as depicted in formula (6) and/or heparan sulfate containing one or more sequences of residues as depicted in formula (6) (Fig. 3) which is capable of binding chemokines CXCL1, CXCL2, CCL2.

In another preferred embodiment, ND1-ΔMD gene, HS3ST5-ΔMD, and HS6ST1-ΔMD is additionally integrated into the genome of the recombinant strain ST_GNS-016, thereby yielding a subsequent recombinant yeast strain ST_GNS-029 that is capable of producing optimised (increased) cytosolic concentration of N-, 6-O-sulfated heparan sulfate as depicted in formula (7) and/or heparan sulfate containing one or more sequences of residues as depicted in formula (7) (Fig. 3) which is capable of binding chemokines of class A, B, C, and D.

### Description of the figures

Fig. 1a: Modular pathway engineering approach to achieve high cytosolic concentrations of UDP-D-glucuronic acid (UDP-GlcA), UDP-acetyl-D-glucosamine (UDP-GlcNAc) and 3'-phospho-adenylylsulfate (PAPS). (e) marks extracellular and (c) marks cytosolic location of D-glucose and sulfate.
Fig. 1b: Chassis optimisation of the yeast for production of UDP-D-glucuronic acid and UDP-N-acetyl-D-glucosamine is necessary for efficient production of glycosaminoglycans.
Fig. 2a: Biosynthesis of heparan sulfate from heparosan.
Fig. 2b: Substrate specificity HS3ST isoforms 1, 3 and 5 mediated heparan sulfate 3-O-sulfate transfer. Legend as in Fig. 2a.
Fig. 3: Distinct heparan sulfates defined by modifications and sulfation patterns have different biological functions. For modifications, NS denotes N-sulfated heparan sulfate, NH2 denotes deacetylated heparan sulfate, 2S denotes 2-O-sulfated heparan sulfate, 3S denotes 3-O-sulfated heparan sulfate, 6S denotes 6-O-sulfated heparan sulfate and C5-epi denotes epimerization of glucuronic acid residues to iduronic acid residues.
Fig. 4: Cloning method to achieve stable gene integration into the yeast genome. The integrative vector is linearized by restriction digestion. Co-transformation of the linearized integrative vector with the helper vector containing the gRNA for the targeted locus into a yeast containing a Cas9 expression vector enables stable gene integration through Cas9 mediated double stranded DNA break and subsequent homology directed repair.

### Material and Methods

### Strains and media

E. coli strain DH5α was used to clone, propagate and store plasmids. Cultivation of E. coli was carried out in Lysogeny Broth (LB) liquid media containing 100 mg/L ampicillin with shaking at 250 rpm and 37 °C. For solid LB-plates, the media was supplemented with 20 g/L agar.

Saccharomyces cerevisiae S288C containing an episomal Cas9 expression vector with a kanamycin resistance marker was used as parent strain for yeast strain construction. Yeast Peptone Dextrose (YPD) Media consisting of 10 g/L yeast extract, 20 g/L peptone and 20 g/L glucose, supplemented with 200 mg/L G418 to ensure maintenance of the Cas9 expression vector was used for yeast culturing at 30 °C and 250 rpm. YPD agar plates were prepared by addition of 20 g/L agar.

### Plasmid construction

The integrative vectors as well as gRNA helper vectors are listed in the Plasmid list, which is disclosed elsewhere herein. The vectors were constructed from PCR products using Gibson assembly or the AQUA cloning protocol. Gibson assembly was performed by incubation of 100 ng of backbone (fragment carrying the origin of replication) and insert in a molar 1:2-3 backbone:insert ratio in a 20 µl reaction volume containing 2X NEB Gibson Assembly Mastermix at 50 °C for 60 minutes.

Similarly, AQUA cloning was performed by incubation of 100 ng of backbone and insert in a molar 1:2-3 backbone:insert ratio in a 10 µl aqueous reaction volume at room temperature for 60 minutes.

Chemical transformation was achieved by adding 2 µl of Gibson assembly or 5 µl of the AQUA cloning mix to 50 µl chemical competent E. coli cells and incubating the cells on ice for 30 minutes. The cells were then heat-shocked at 42 °C for 30 seconds and subsequently mixed with 950 µl LB media. Transformed cells were plated on LB-agar plates containing ampicillin. Following overnight incubation at 37 °C, colonies were screened by colony PCR for correct Plasmid construction using Promega's GoTaq G2 DNA Polymerase according to the manufacturer's protocol.

The fragments ("biobricks") for plasmid construction were obtained by PCR. PCR-reactions with plasmids, genomic DNA or synthetic gene fragments as templates were performed using Q5 High-Fidelity DNA Polymerase from NEB according to the manufacturer's protocol.

All heterologous genes were codon optimised for expression in S. cerevisiae and synthesized as synthetic gene fragments by Twist Bioscience.

To ensure gene expression in the yeast, the codon optimised genes where cloned to be preceded by a promoter, preferably a strong promoter at their 5'-end and followed by a terminator at their 3'-end (Fig. 4). This expression cassette (promoter, gene, terminator) or a number of these expression cassettes joined together where cloned to be flanked by homology arms to ensure stable chromosomal integration in the yeast. For example, the construct for the expression of two genes may consist of (Fig. 4):
(1) Upstream homology arm (i.e. 5'-homology arm) (ca. 500 bp)
(2) ADH1-terminator (tADH1)
(3) Gene 1
(4) TDH3-promoter (pTDH3)
(5) TEF1-promoter (pTEF1)
(6) Gene 2
(7) CYC1-terminator (tCYC1)
(8) Downstream homology arm (i.e. 3'-homology arm) (ca. 500 bp)

The constructs (5'-homology arm, expression cassette(s), 3'-homology arm) were designed to be on a plasmid that also expresses an ampicillin resistance marker and an origin of replication (ori) to ensure plasmid maintenance in E. coli. To facilitate linearisation of the construct (5'-homology arm, expression cassette(s), 3'-homology arm), the plasmid was designed to contain Not1 restriction sites on either side.

### Strain construction

Stable chromosomal gene integration into the yeast was achieved using a toolkit of integrative vectors and helper vectors containing the corresponding gRNAs. Before transforming, the integrative vector for a certain chromosomal locus containing the heterologous gene construct (containing e.g. promoter, gene, terminator) flanked by the up- and downstream homology arms (ca. 500 bp long each) was linearized by restriction enzyme (Notl) digestion. Digestion was carried out according to the digestion enzyme manufacturer's protocol. 1.5-3 µg of linearized expression cassette was subsequently transformed into the Cas9 expressing parental strain together with 200-500 ng of helper vector, expressing the corresponding gRNA for the targeted locus as well as a nourseothricin resistance marker (Fig. 4). The transformations were performed according to the standard lithium acetate transformation method. The transformed cells were plated on YPD plates containing 200 mg/L G418 and 100 mg/L nourseothricin. Transformants were verified by colony PCR, using Promega's GoTaq G2 DNA Polymerase according to the manufacturer's protocol, and plasmid-cured to remove the gRNA vector. Curing the strain of the gRNA helper vector was achieved by growing the strain in liquid YPD supplemented with 200 mg/L G418 but without nourseothricin for 12 hours. After curing, the strain is ready for the next round of stable chromosomal gene integration. Stable gene integration, in particular marker-free stable gene integration (in contrast to integration of plasmids for gene expression) does not require co-expression of markers (e.g. antibiotic resistance genes) for gene maintenance.

### Stable Integration Technique for Strain Construction

Stable and marker-free integration of recombinant DNA into the yeast genome was achieved using CRISPR/Cas9 induced homology directed repair as described in the following: The recombinant DNA that is to be integrated (often an expression cassette consisting of promoter, gene and terminator) is cloned to be flanked by homology arms (upstream homology arm denoted by UP is the homology arm at the 5' end of the construct, downstream homology arm denoted by DOWN is the homology arm at the 3' end of the construct) (Fig 4). The homology arms (about 500 bp long each) consist of the DNA-sequence identical to the DNA-sequence down- and upstream of the desired integration locus (Fig 4). The linearised DNA-fragment consisting of 5'-homology arm, recombinant DNA that is to be integrated and the 3'-homology arm (in that order) is then transformed into the the parent strain together with a plasmid that expresses the guide RNA (gRNA) that corresponds to the targeted locus of the homology arms of the linearised DNA-fragment. The plasmid expressing the gRNA (helper gRNA vector) carries a natMX6 nourseothricin resistance marker to ensure the maintenance of the plasmid in the yeast. The expressed gRNA consists of 20 variable basepairs (depending on targeted locus) at its 5'-end and the gRNA scaffold sequence at the 3'-end (5'-GUUUUAGAGCUAGAAAUAGCAAGUUAAAAUAAGGCUAGUCCGUUAU CAACUUGAAAAAGUGGCACCGAGUCGGUG-3'). The parent yeast strain into which the linearised DNA-fragment and the helper gRNA vector (plasmid) are simultaneously introduced by transformation also carries a plasmid expressing the endonuclease Cas9 as well as a KanMX kanamycin resistance marker to ensure plasmid maintenance. The expressed Cas9 is directed to the targeted chromosomal locus by the expressed gRNA (from the helper gRNA vector) where the endonuclease induces a double strand break. The double strand break induces the homology directed repair mechanism of the yeast cell, which uses the linearised DNA-fragment (due to its homology arms matching the up- and down-stream regions of the induced double strand break) as repair template, thereby integrating the recombinant DNA-fragment that is to be integrated.

A list of the generated yeast strains is disclosed elsewhere herein.

### Production and purification

The production strains were grown in shake flasks for 96 hours at 250 rpm and 30 °C. The cultures was then spun down at 12,000 g for 30 minutes at 4 °C. Subsequently, the recovered supernatant was adjusted to pH 4 using acetic acid and then filtered through a fritted filter funnel of pore size 60 µm. For purification, a column packed with Sepharose fast flow resin was first equilibrated with buffer A. Buffer A consists of 50 mM sodium chloride and 20 mM sodium acetate at pH 4. Following equilibration, the filtered culture supernatant was loaded onto the column and washed with three volumes of buffer A. The product was then eluted from the column using buffer B consisting of 1 M sodium chloride and 20 mM sodium acetate at pH 4. The product was then precipitated by addition of three volumes of ethanol and spun down at 12,000 g for 30 minutes. The pellet was washed using 75% ethanol and again spun down at 12,000 g for 30 minutes to obtain the purified product.

### Product analysis

For analysis, the product was dissolved in a 200 mM sodium phosphate buffer at pH 7 and digested at 30 °C for 12 hours with 2 units of heparinase I and III blend from Flavobacterium heparinum obtained from Sigma-Aldrich. The resulting disaccharides were sent to Eurofins Scientific SE for LC-MS analysis.

### Precursor Analysis

For quantification of precursor-production (i.e. production of UDP-D-glucuronic acid, UDP-N-acetyl-D-glucosamine and 3'-phospho-adenylylsulfate), the fermentation broth was centrifuged at 10,000 g for 5 minutes. Ultrasonic disruption can be used prior to the centrifugation step if cellular metabolite concentrations need to be determined. After centrifugation, the supernatant was transferred to amber vials for HPLC analysis. Samples were then analysed by HPLC using a reverse-phase column. Standards for the quantification of the metabolites by HPLC can be obtained from Sigma-Aldrich (product ID for UDP-glucuronic acid: U6751-500MG, product ID for UDP-N-acetyl: U4375-500MG, product ID for 3'-phospho-adenylylsulfate: 94455-25MG).

### Transcriptional Analysis

In order to determine the mRNA level, the following protocol can be used: About 10^7 cells need to be selected for RNA extraction using a coulter counter. RNA extraction for transcriptional analysis can then be achieved using a commercial kit (e.g. NEB Monarch Total RNA Miniprep Kit) according to the manufacturer's protocol. Real-time quantitative PCR (RT-qPCR) can then be performed in 96-well plates using a Biorad ICycler and SYBR-Green according to the manufacturer's protocol.

### References

1. Oka T., Jigami Y., Reconstruction of de novo pathway for synthesis of UDP-glucuronic acid and UDP-xylose from intrinsic UDP-glucose in Saccharomyces cerevisiae. The FEBS Journal 2006 Jun; 273(12):2645-57. doi: 10.1111/j.1742-4658.2006.05281.x.
2. Badri, A., Williams, A., Xia, K., Linhardt, R., Koffas, M. Increased 3'-phosphoadenosine-5'-phosphosulfate levels in engineered Escherichia coli cell lysate facilitates in vitro synthesis of Chondroitin Sulfate A. Biotechnology Journal 2019 Sep;14(9):e1800436. doi: 10.1002/biot.201800436.
3. Williams, A., Gedeon, K., Vaidyanathan, D.,Yu, Y., Collins, C., Dordick, J., Linhardt, R., Koffas. M.Metabolic engineering of Bacillus megaterium for heparosan biosynthesis using Pasteurella multocida heparosan synthase, PmHS2. Microbial Cell Factories 2019Aug 12;18(1):132. doi: 10.1186/s12934-019-1187-9.
4. Xu, D., Moon, A., Song, D., Pedersen, L., Liu, J. Engineering Sulfotransferases to Modify Heparan Sulfate. Nature Chemical Biology 2008 Mar;4(3):200-2. doi: 10.1038/nchembio.66. Epub 2008 Jan 27.
5. Mah, D., Zhao, J., Liu, X., Zhang, F., Liu, J., Wang, L., Linhardt, R., Wang, C. The Sulfation Code of Tauopathies- Heparan Sulfate Proteoglycans in the Prion Like Spread of Tau Pathology. Frontiers in Molecular Biosciences 2021 May 20;8:671458. doi: 10.3389/fmolb.2021.671458.

**Sequence listing:**

| **SEQ ID NO** | **Description** |
|---|---|
| 1 | protein sequence of UDP-D-glucose dehydrogenase 1 from Arabidopsis thaliana, AtUGD1 |
| 2 | S. cerevisiae codon-optimised nucleotide sequence of AtUGD1 from Arabidopsis thaliana |
| 3 | protein sequence of heparosan synthase 1 from Pasteurella multocida, PmHS1 |
| 4 | S. cerevisiae codon-optimised nucleotide sequence of PmHS1 from Pasteurella multocida |
| 5 | protein sequence of heparosan synthase 2 from Pasteurella multocida, PmHS2 |
| 6 | S. cerevisiae codon-optimised nucleotide sequence of PmHS2 from Pasteurella multocida |
| 7 | protein sequence of heparosan synthase 1 from Pasteurella multocida fused to a nuclear export sequence, PmHS1_NES |
| 8 | S. cerevisiae codon-optimised nucleotide sequence of PmHS1_NES |
| 9 | protein sequence of heparosan synthase 2 from Pasteurella multocida fused to a nuclear export sequence, PmHS2_NES |
| 10 | S. cerevisiae codon-optimised nucleotide sequence of PmHS2_NES |
| 11 | protein sequence of N-deacetylase-N-sulfotransferase 1 from Mus musculus, NDST1 without N-terminal membrane domain |
| 12 | S. cerevisiae codon-optimised nucleotide sequence of NDST1 from Mus musculus without N-terminal membrane domain |
| 13 | protein sequence of D-glucuronyl C5-epimerase from Homo sapiens, C5-epi without N-terminal membrane domain |
| 14 | S. cerevisiae codon-optimised nucleotide sequence of C5-epi from Homo sapiens without N-terminal membrane domain |
| 15 | protein sequence of heparan sulfate 2-O-sulfotransferase 1 from Homo sapiens, HS2ST1 without N-terminal membrane domain |
| 16 | S. cerevisiae codon-optimised nucleotide sequence of HS2ST1 from Homo sapiens without N-terminal membrane domain |
| 17 | protein sequence of heparan sulfate 3-O-sulfotransferase 1 from Mus musculus, HS3ST1 without N-terminal signal domain/signal peptide |
| 18 | S. cerevisiae codon-optimised nucleotide sequence of HS3ST1 from Mus musculus without N-terminal signal domain/singal peptide |
| 19 | protein sequence of heparan sulfate 3-O-sulfotransferase 3 A1 from Homo sapiens, HS3ST3 A1 without N-terminal membrane domain |
| 20 | S. cerevisiae codon-optimised nucleotide sequence of HS3ST3 A1 from Homo sapiens without N-terminal membrane domain |
| 21 | protein sequence of heparan sulfate 3-O-sulfotransferase 5 from Homo sapiens, HS3ST5 without N-terminal membrane domain |
| 22 | S. cerevisiae codon-optimised nucleotide sequence of HS3ST5 from Homo sapiens without N-terminal membrane domain |
| 23 | protein sequence of heparan sulfate 6-O-sulfotransferase 1 from Mus musculus, HS6ST1 without N-terminal membrane domain |
| 24 | S. cerevisiae codon-optimised nucleotide sequence of HS6ST1 from Mus musculus without N-terminal membrane domain |
| 25 | protein sequence of phosphoglucomutase 1 from Saccharomyces cerevisiae, PGM1 |
| 26 | nucleotide sequence of phosphoglucomutase 1 from Saccharomyces cerevisiae, PGM1 |
| 27 | protein sequence of phosphoglucomutase 2 from Saccharomyces cerevisiae, PGM2 |
| 28 | nucleotide sequence of phosphoglucomutase 2 from Saccharomyces cerevisiae, PGM2 |
| 29 | protein sequence of UDP-glucose pyrophosphorylase 1 from Saccharomyces cerevisiae, UGP1 |
| 30 | nucleotide sequence of UDP-glucose pyrophosphorylase 1 from |
| | Saccharomyces cerevisiae, UGP1 |
| 31 | protein sequence of glutamine-fructose-6-phosphate amidotransferase 1 from Saccharomyces cerevisiae, GFA1 |
| 32 | nucleotide sequence of glutamine-fructose-6-phosphate amidotransferase 1 from Saccharomyces cerevisiae, GFA1 |
| 33 | protein sequence of phosphoacetylglucosamine mutase 1 from Saccharomyces cerevisiae, PCM1 |
| 34 | nucleotide sequence of phosphoacetylglucosamine 1 mutase from Saccharomyces cerevisiae, PCM1 |
| 35 | protein sequence of methionine requiring protein 3 from Saccharomyces cerevisiae, MET3 |
| 36 | nucleotide sequence of methionine requiring protein 3 from Saccharomyces cerevisiae, MET3 |
| 37 | protein sequence of methionine requiring protein 14 from Saccharomyces cerevisiae, MET14 |
| 38 | nucleotide sequence of methionine requiring protein 14 from Saccharomyces cerevisiae, MET14 |
| 39 | protein sequence of methionine requiring protein 22 from Saccharomyces cerevisiae, MET22 |
| 40 | nucleotide sequence of methionine requiring protein 22 from Saccharomyces cerevisiae, MET22 |
| 41 | protein sequence of chitin-synthase 2 from Saccharomyces cerevisiae, CHS2 |
| 42 | nucleotide sequence of chitin-synthase 2 from Saccharomyces cerevisiae, CHS2 |
| 43 | protein sequence of chitin-synthase 3 from Saccharomyces cerevisiae, CHS3 |
| 44 | nucleotide sequence of chitin-synthase 3 from Saccharomyces cerevisiae, CHS3 |
| 45 | protein sequence of 3'-phosphoadenylyl-sulfate reductase from Saccharomyces cerevisiae, MET16 |
| 46 | nucleotide sequence of 3'-phosphoadenylyl-sulfate reductase from Saccharomyces cerevisiae, MET16 |
| 47 | nucleotide sequence of native CHS3 promoter from Saccharomyces cerevisiae, pCHS3 |
| 48 | nucleotide sequence of native MET16 promoter from Saccharomyces cerevisiae, pMET16 |
| 49 | nucleotide sequence of native KEX2 promoter from Saccharomyces cerevisiae, pKEX2 |
| 50 | nucleotide sequence of native TDH3 promoter from Saccharomyces cerevisiae, pTDH3 |
| 51 | nucleotide sequence of native TEF1 promoter from Saccharomyces cerevisiae, pTEF1 |
| 52 | nucleotide sequence of native PGK1 promoter from Saccharomyces cerevisiae, pPGK1 |
| 53 | protein sequence of the C-terminal nuclear export sequence from Saccharomyces cerevisiae, NES |
| 54 | nucleotide sequence of the C-terminal nuclear export sequence from Saccharomyces cerevisiae, NES |
| 55 | nucleotide sequence of Hsp70-like chaperone from Saccharomyces cerevisiae, SSB1 |
| 56 | protein sequence of Hsp70-like chaperone from Saccharomyces cerevisiae, SSB1 |
| 57 | protein sequence of N-deacetylase 1 from Mus musculus, ND1 without N-terminal membrane domain |
| 58 | S. cerevisiae codon-optimised nucleotide sequence of ND1 from Mus musculus without N-terminal membrane domain |
| 59 | nucleotide sequence of the guide RNA (gRNA) scaffold, artificially synthetic nucleotide sequence |

**Plasmid list**

| **Name** | **Description** | **Purpose** |
|---|---|---|
| pGNS_ZER O_013 | gRNA for Cas9 targeting Chr X: 223616..224744 | helper (gRNA) vector |
| pGNS_ZER O_014 | gRNA for Cas9 targeting Chr X: 236336..237310 | helper (gRNA) vector |
| pGNS_ZER O_016 | gRNA for Cas9 targeting Chr XI: 91575..92913 | helper (gRNA) vector |
| pGNS_ZER O_017 | gRNA for Cas9 targeting Chr XI: 93378..94567 | helper (gRNA) vector |
| pGNS_ZER O_018 | gRNA for Cas9 targeting Chr XI: 11779..118967 | helper (gRNA) vector |
| pGNS_ZER O_019 | gRNA for Cas9 targeting Chr XII: 795787..796720 | helper (gRNA) vector |
| pGNS_ZER O_020 | gRNA for Cas9 targeting Chr XII: 808805..809939 | helper (gRNA) vector |
| pGNS_ZER O_021 | gRNA for Cas9 targeting Chr XII:830227 ..831248 | helper (gRNA) vector |
| pGNS_ZER O_022 | gRNA for Cas9 targeting Chr XII: 839226..840357 | helper (gRNA) vector |
| pGNS_ZER O_049 | integrative vector with pTDH3_pTEF1 promoter cassette | used for amplificatin of pTDH3_pTEF1 promoter cassette |
| pGNS_ON E-001 | Chr X: 236336..237310::tADH1_AtUGD1_pTDH3_ pTEF1_pmHS1_tCYC1 | integration vector for AtUGD1 and PmHS1 |
| pGNS_ON E-002 | Chr X: 236336..237310::tADH1_AtUGD1_pTDH3_ pTEF1_pmHS2_tCYC1 | integration vector for AtUGD1 and PmHS2 |
| pGNS_ON E-003 | Chr XI: 91575..92913::tADH1_AtUGD1_pTDH3_pT EF1_pmHS1_tCYC1 | integration vector for AtUGD1 and PmHS1 |
| pGNS_ON E-004 | Chr X: 236336..237310::tADH1_AtUGD1_pTDH3_ pTEF1_pmHS1_NES_tCYC1 | integration vector for AtUGD1 and PmHS1_NES |
| pGNS_ON E-005 | Chr X: 236336..237310::tADH1_AtUGD1_pTDH3_ pTEF1_pmHS2_NES_tCYC1 | integration vector for AtUGD1 and PmHS2_NES |
| pGNS_ON E-006 | Chr XI: 93378..94567::tADH1_NDST1-ΔMD_pTDH3_pTEF1_C5-epimerase-ΔMD_tCYC1 | integration vector for NDST1-ΔMD and C5-epimerase-ΔMD |
| pGNS_ON E-007 | Chr X: 236336..237310::tADH1_AtUGD1_pTDH3_ pTEF1_tCYC1 | integration vector for AtUGD1 |
| pGNS_ON E-010 | Chr XI: 11779..118967::tADH1_GFA1_pTDH3_pTE F1_PCM1_tCYC1 | integration vector for GFA1 and PGM1 overexpression |
| pGNS_ON E-011 | Chr XII: 795787..796720::tADH1_UGP1_pTDH3_pT EF1_PGM2_tCYC1 | integratin vector for UGP1 and PGM2 overexpression |
| pGNS_ON E-012 | Chr II: 287926..288866::3'overhang_pKEX2_5'CHS 3 - CHS3 promoter switch | integration vector for promoter switch for CHS3 promoter |
| pGNS_ON E-017 | Chr XI: 91575..92913::tADH1_AtUGD1_pTDH3_pT EF1_pmHS1_NES_tCYC1 | integration vector for AtUGD1 and PmHS1_NES |
| pGNS_ON E-018 | Chr XI: 93378..94567::tADH1_NDST1-ΔMD_pTDH3_pTEF1_tCYC1 | integration vector for NDST1-ΔMD |
| pGNS_ON E-019 | Chr XI: 11779..118967::tADH1_AtUGD1_pTDH3_p TEF1_pmHS1_tCYC1 | integration vector for AtUGD1 and PmHS1 |
| pGNS_ON E-020 | Chr XI: 11779..118967::tADH1_AtUGD1_pTDH3_p TEF1_pmHS1_NES_tCYC1 | integration vector for AtUGD1 and PmHS1_NES |
| pGNS_ON E-021 | Chr XII: 808805..809939::tADH1 _ HS2ST1-ΔMD_pTDH3_pTEF1 | integration vector for HS2ST1-ΔMD |
| pGNS_ON E-022 | Chr XII: 808805..809939::tADH1 _ HS2ST1-ΔMD_pTDH3_pTEF1_HS6ST1-ΔMD_tCYC1 | integration vector for HS2ST1-ΔMD and HS6ST1-ΔMD |
| pGNS_ON E-023 | Chr XII:830227..831248::tADH1_HS3ST1-ΔSD_pTDH3_pTEF1 | integration vector for HS3ST1-ΔSD |
| pGNS_ON E-024 | Chr XII:830227..831248::tADH1_HS3ST3 A1-ΔMD_pTDH3_pTEF1 | integration vector for HS3ST3-ΔMD |
| pGNS_ON E-025 | Chr XII:830227..831248::tADH1_HS3ST5-ΔMD_pTDH3_pTEF1 | integration vector for HS3ST5-ΔMD |
| pGNS_ON E-026 | Chr XII: 839226..840357::tADH1_MET3_pTDH3_pT EF1_MET14_tCYC1 | integration vector for MET3 and MET14 overexpression |
| pGNS_ON E-027 | Chr XII: 839226..840357::tADH1_MET3_pTDH3_pT EF1_MET14_tCYC1_tPRM9_MET22_pPGK 1 | integration vector for MET3, MET14 and MET22 overexpression |
| pGNS_ON E-028 | gRNA for Cas9 targeting CHS3 promoter | helper (gRNA) vector for CHS3 promoter switch |
| pGNS_ON E-029 | Chr XVI: 877022..878396::5'MET16_pKEX2_3'overha ng - MET16 promoter switch | integration vector for promoter switch for MET16 promoter |
| pGNS_ON E-030 | gRNA for Cas9 targeting MET16 promoter | helper (gRNA) vector for MET16 promoter switch |
| pGNS_ON E-031 | Chr X: 223616..224744::tADH1_PmHS1_NES_pTD H3_pTEF1_PmHS2_NES_tCYC1 | integration vector for PmHS1_NES and PmHS2_NES |
| pGNS_ON E-032 | Chr XII: 808805..809939::tADH1 _ HS6ST1-ΔMD_pTDH3_pTEF1 | integration vector for HS6ST1 |

**Yeast strain list**

| **Strain name** | **Parent strain** | **Integrated DNA Element** | **Description** |
|---|---|---|---|
| ST_GNS-001 | S. cerevisiae S288C with Cas9 episomal vector | Chr X: 236336..237310::tADH1_AtUG D1_pTDH3_pTEF1_pmHS1_tC YC1 | AtUGD1 and PmHS1 expressing strain for heparosan biosynthesis |
| ST_GNS-002 | S. cerevisiae S288C with Cas9 episomal vector | Chr X: 236336..237310::tADH1_AtUG D1_pTDH3_pTEF1_pmHS2_tC YC1 | AtUGD1 and PmHS2 expressing strain for heparosan biosynthesis |
| ST_GNS-003 | S. cerevisiae S288C with Cas9 episomal vector | Chr X: 236336..237310::tADH1_AtUG D1_pTDH3_pTEF1_pmHS1_N ES_tCYC1 | AtUGD1 and PmHS1_NES expressing strain for heparosan biosynthesis |
| ST_GNS-004 | S. cerevisiae S288C with Cas9 episomal vector | Chr X: 236336..237310::tADH1_AtUG D1_pTDH3_pTEF1_pmHS2_N ES_tCYC1 | AtUGD1 and PmHS2_NES expressing strain for heparosan biosynthesis |
| ST_GNS-005 | S. cerevisiae S288C with Cas9 episomal vector | Chr X: 236336..237310::tADH1_AtUG D1_pTDH3_pTEF1_tCYC1 | AtUGD1 expressing strain for UDP-D-glucuronic acid biosynthesis |
| ST_GNS-006 | ST_GNS-002 | Chr XI: 93378..94567::tADH1_NDST1-ΔMD_pTDH3_pTEF1_C5-epimerase-ΔMD_tCYC1 | strain producing heparan-N-sulfate with iduronic acid residues |
| ST_GNS-007 | ST_GNS-004 | Chr XI: 91575..92913::tADH1_AtUGD1 _pTDH3_pTEF1_pmHS1_NES tCYC1 | strain producing heparosan |
| ST_GNS-008 | S. cerevisiae S288C with Cas9 episomal vector | Chr XI: 11779..118967::tADH1_GFA1_ pTDH3_pTEF1_PCM1_tCYC1 | strain overexpressing endogenous UDP-acetyl-D-glucosamine biosynthesis pathway |
| ST_GNS-009 | ST_GNS-005 | Chr XII: 795787..796720::tADH1_UGP1 _pTDH3_pTEF1_PGM2_tCYC 1 | strain overexpressing endogenous UDP-D-Glucose biosynthesis pathway |
| ST_GNS-010 | ST_GNS-009 | Chr XI: 11779..118967::tADH1_GFA1_ pTDH3_pTEF1_PCM1_tCYC1 | strain overexpressing endogenous UDP-acetyl-D-glucosamine biosynthesis pathway and endogenous UDP-D-Glucose biosynthesis pathway |
| ST_GNS-011 | ST_GNS-010 | Chr II: 287926..288866::3'overhang_p KEX2_5'CHS3 - CHS3 promoter switch | UDP-precursor optimised strain |
| ST_GNS-012 | ST_GNS-011 | Chr XII: 839226..840357::tADH1_ MET3 _pTDH3_pTEF1_MET14_tCYC 1 | UDP-precu rsor optimised strain, additional overexpression of PAPS biosynthesis pathway |
| ST_GNS-013 | ST_GNS-011 | Chr XII: 839226..840357::tADH1_MET3 _pTDH3_pTEF1_MET14_tCYC 1 tPRM9 _ MET22_pPGK1 | UDP-precu rsor and PAPS optimised strain (i.e. precursor optimised strain) |
| ST_GNS-014 | ST_GNS-013 | Chr XVI: 877022..878396::5'MET16_pK EX2_3'overhang - MET16 promoter switch | UDP-precu rsor and PAPS optimised strain (i.e. precursor optimised strain) |
| ST_GNS-015 | ST_GNS-014 | Chr X: 236336..237310::tADH1_AtUG D1_pTDH3_pTEF1_pmHS2_N ES_tCYC1 | integeration of PmHS2_NES in precursor optimised strain |
| ST_GNS-016 | ST_GNS-015 | Chr XI: 91575..92913::tADH1_AtUGD1 _pTDH3_pTEF1_pmHS1_NES tCYC1 | integeration of PmHS2_NES, PmHS1_NES in precursor optimised strain |
| ST_GNS-017 | ST_GNS-007 | Chr XI: 93378..94567::tADH1_NDST1-ΔMD_pTDH3_pTEF1_C5-epimerase-AMD_tCYC1 | strain producing heparan-N-sulfate with iduronic acid residues |
| ST_GNS-018 | ST_GNS-017 | Chr XII: 808805..809939::tADH1_HS2S T1-ΔMD_pTDH3_pTEF1_HS6ST1 -ΔMD_tCYC1 | strain producing N,2,6-O sulfated heparan sulfate |
| ST_GNS-019 | ST_GNS-018 | XII-4::tADH1_HS3ST1-ΔSD_pTDH3_pTEF1 | strain producing N,2,3,6-O sulfated heparan sulfate |
| ST_GNS-020 | ST_GNS-016 | Chr XI: 93378..94567::tADH1_NDST1-ΔMD_pTDH3_pTEF1_C5-epimerase-ΔMD_tCYC1 | strain producing heparan-N-sulfate with iduronic acid residues |
| ST_GNS-021 | ST_GNS-020 | Chr XII: 808805..809939::tADH1 _HS2S T1-ΔMD_pTDH3_pTEF1_HS6ST1 -ΔMD_tCYC1 | strain producing N,2,6-O sulfated heparan sulfate |
| ST_GNS-022 | ST_GNS-021 | Chr XII:830227..831248::tADH1_H S3ST1-ΔS D_pTDH 3_pTE F1 | strain producing N,2,3,6-O sulfated heparan sulfate |
| ST_GNS-023 | ST_GNS-021 | Chr XII:830227..831248::tADH1_H S3ST3 A1-ΔMD_pTDH3_pTEF1 | strain producing N,2,3,6-O sulfated heparan sulfate |
| ST_GNS-024 | ST_GNS-021 | Chr XII:830227..831248::tADH1_H S3ST5-ΔMD_pTDH3_pTEF1 | strain producing N,2,3,6-O sulfated heparan sulfate |
| ST_GNS-025 | ST_GNS-016 | Chr XI: 93378..94567::tADH1 _ NDST1-ΔMD_pTDH3_pTEF1 | strain prouducing N-sulfated heparan sulfate |
| ST_GNS-026 | ST_GNS-025 | Chr XII: 808805..809939::tADH1_HS6S T1-ΔMD_pTDH3_pTEF1 | strain producing N,6-sulfated heparan sulfate |
| ST_GNS-027 | ST_GNS-026 | Chr XII:830227..831248::tADH1_H S3ST5-ΔMD_pTDH3_pTEF1 | strain producing N,3,6-sulfated heparan sulfate |
| ST_GNS-028 | ST_GNS-020 | Chr XII: 808805..809939::tADH1 _ HS6S T1-ΔMD_pTDH3_pTEF1 | strain prouducing N,6-sulfated heparan sulfate with iduronic acid residues |
| | | Chr XI: 93378..94567::tADH1 _ ND1-ΔMD_pTDH3_pTEF1_tCYC1, | |
| ST_GNS-029 | ST_GNS-016 | Chr XII: 808805..809939::tADH1 _ HS6S T1-ΔMD_pTDH3_pTEF1, Chr | |
| | | XII:830227..831248::tADH1_H S3ST5-ΔMD_pTDH3_pTEF1 | strain producing NH2,3,6-sulfated heparan sulfate |

## Claims

1. Recombinant yeast cell, **characterized in that** it produces an increased amount of UDP-GlcA (UDP-D-glucuronic acid) and/or UDP-GlcNAc (UDP-N-acetyl-D-glucosamine); and optionally PAPS (3'-phospho-adenylylsulfate), when compared to its wildtype cell.

2. Recombinant yeast cell according to claim 1, **characterized in that** it expresses an increased amount of one or more enzymes selected from the group consisting of: UTP-glucose-1-phosphat-uridylyltransferase (UDP-glucose pyrophosphorylase), phosphoglucomutase, glutamine-fructose-6-phosphate transaminase, N-acetyl-glucosamine-phosphate mutase; and optionally ATP-sulfurylase, adenylyl-sulfate kinase, and bisphosphate-3'-nucelotidase, when compared to its wildtype cell.

3. Recombinant yeast cell according to claim 1, **characterized in that**
•
(i) one or more of the genes that are involved in UDP-GlcA synthesis; and/or
(ii) one or more of the genes that are involved in UDP-GlcNAc synthesis; and optionally
(iii) one or more of the genes that are involved in PAPS synthesis are overexpressed; and
• a gene that encodes heterologous UDP-glucose dehydrogenase is expressed; thereby resulting in an increased amount of UDP-GlcA, UDP-GlcNAc, and optionally PAPS, when compared to the expression of the respective gene(s) in its wildtype cell.

4. Recombinant yeast cell according to claim 3, **characterized in that**
• the genes that are involved in (i) UDP-GlcA synthesis are genes that encode phosphoglucomutase, preferably Phosphoglucomutase 1/2 (PGM1/2); and UTP-glucose-1-phosphat-uridylyltransferase (UDP-glucose pyrophosphorylase), preferably UDP-glucose pyrophosphorylase 1 (UGP1);
• the genes that are involved in (ii) UDP-GlcNAc synthesis are genes that encode glutamine-fructose-6-phosphate transaminase, preferably Glutamine-fructose-6-phosphate amidotransferase 1 (GFA1); and N-acetyl-glucosamine-phosphate mutase, preferably Phosphoacetylglucosamine Mutase 1 (PCM1); and/or the genes that are involved in (iii) PAPS synthesis are genes that encode sulfate adenylyltransferase (ATP-sulfurylase), preferably METhionine requiring protein 3 (MET3); adenylyl-sulfate kinase, preferably METhionine requiring protein 14 (MET14); and bisphosphate-3'-nucleotidase, preferably METhionine requiring protein 22 (MET22).

5. Recombinant yeast cell according to any one of claims 1 to 4, **characterized in that**
• a gene encoding the enzyme chitin-synthase, preferably chitin-synthase (CHS2/3), is downregulated, to the effect that the amount of the chitin-synthase is decreased; and/or
• a gene encoding the enzyme 3'-phosphoadenylylsulfate reductase, preferably MET16, is downregulated,
to the effect that the amount of the respective enzyme the down-regulated gene encodes is decreased, when compared to its wildtype cell.

6. Recombinant yeast cell according to any one of claims 1 to 5, **characterized in that** it additionally expresses gene(s) encoding a heparosan synthase, preferably Heparosan synthase 1 and/or Heparosan synthase 2, to the effect that the recombinant yeast cell is able to produce, preferably produces, heparosan.

7. Recombinant yeast cell according to claim 6, **characterized in that** it additionally expresses gene(s) encoding N-deacetylase (ND), and/or N-deacetylase-N-sulfotransferase (NDST), and/or C5-epimerase (C5-epi), and/or 2-O-sulfotransferase (HS2ST), and/or 3-O-sulfotransferase (HS3ST), and/or 6-O-sulfotransferase (HS6ST), thereby being capable of producing heparan sulfates.

8. Recombinant yeast cell according to claim 6, **characterized in that** it additionally expresses gene(s) encoding ND, and/or, NDST, and/or C5-epi, and/or HS2ST, and/or HS3ST, and/or HS6ST truncated to exclude the N-terminal membrane domain (MD) or N-terminal signal peptide/ signal domain (SD), thereby expressing ND, and/or NDST, and/or C5-epi, and/or HS2ST, and/or HS3ST, and/or HS6ST enzymes specifically in cytosol, thereby increasing the production of heparan sulfates with heterogeneously well-defined sulfation patterns and/or heparin.

9. Recombinant yeast cell according to claim 7 or claim 8, **characterized in that**
• genes encoding NDST, C5-epi, HS2ST, HS3ST1 and/or HS3ST1-like, and HS6ST are additionally expressed, thereby being able to produce heparan sulfate as depicted in Formula (1) in Fig. 3, preferably the recombinant yeast strain is ST_GNS-022;
• genes encoding NDST, HS3ST5 and/or HS3ST5-like, and HS6ST are additionally expressed, thereby being able to produce heparan sulfates as depicted in Formula (2) in Fig. 3;
• genes encoding NDST, and HS6ST are additionally expressed, thereby being able to produce heparan sulfates as depicted in Formula (3) in Fig. 3;
• that genes encoding NDST, C5-epi, HS2ST, and HS6ST are additionally expressed, thereby being able to produce heparan sulfates as depicted in Formula (4) in Fig. 3;
• genes encoding NDST, C5-epi, HS2ST, HS3ST1 and/or HS3ST1-like, and HS6ST are additionally expressed, thereby being able to produce heparan sulfate as depicted in Formula (5) in Fig. 3;
• genes encoding NDST, C5-epi, and HS6ST are additionally expressed, thereby being able to produce heparan sulfate as depicted in Formula (6) in Fig. 3;
• genes encoding ND, HS3ST5 and/or HS3ST5-like, and HS6ST are additionally expressed, thereby being able to produce heparan sulfate as depicted in Formula (7) in Fig. 3; and/or
• genes encoding NDST, C5-epi, HS2ST, HS3ST5 and/or HS3ST5-like, and HS6ST are additionally expressed, thereby being able to produce heparan sulfate as depicted in Formula (8) in Fig. 3; and/or
• genes encoding NDST, C5-epi, HS2ST, HS3ST3 and/or HS3ST3-like, and HS6ST are additionally expressed, thereby being able to produce heparan sulfate as depicted in Formula (9) in Fig. 3.

10. A method of producing heparan sulfate, comprising the steps of
(i) cultivating the recombinant yeast cell as defined in any of claims 1 to 9 in a culture medium;
(ii) obtaining the heparan sulfate produced by the recombinant yeast cell of (i); and optionally
(iii) purifying the obtained heparan sulfate of step (ii).

11. Use of a recombinant yeast cell as defined in any of claims 1 to 9 for producing heparan sulfate.

12. Heparan sulfate, **characterized in that** it exhibits a sulfation pattern resulting from the process as defined in claim 10.
